# EUROPÄISCHE PATENTSCHRIFT

(11) **EP 3 994 117 B1**
(45) Veröffentlichungstag und Bekanntmachung des Hinweises auf die Patenterteilung: **03.07.2024**
(21) Anmeldenummer: 20733830.2
(22) Anmeldetag: 24.06.2020
(51) Int. Cl.: C07F 9/576, C07F 15/00, C07C 29/149, C07C 41/26, C07D 213/30, C07C 33/26, C07C 31/27, C07C 33/02, C07C 33/22, C07C 31/08, C07C 31/125, C07C 33/20, C07C 43/23, C07C 31/20

(54) **HYDRIERUNG VON ESTERN ZU ALKOHOLEN IN GEGENWART EINES RU-PNN-KOMPLEXES**
HYDROGENATION OF ESTERS TO ALCOHOLS IN THE PRESENCE OF A RU-PNN COMPLEX
HYDROGÉNATION D'ESTERS EN VUE D'OBTENIR DES ALCOOLS EN PRÉSENCE D'UN COMPLEXE DE RU-PNN

(30) Priorität: 03.07.2019 EP 19184178; 29.04.2020 EP 20171976
(43) Veröffentlichungstag der Anmeldung: 11.05.2022
(73) Patentinhaber: BASF SE, 67056 Ludwigshafen am Rhein (DE)
(72) Erfinder: SCHELWIES, Mathias, 67056 Ludwigshafen (DE); SCHWABEN, Jonas, 67056 Ludwigshafen (DE); PACIELLO, Rocco, 67056 Ludwigshafen (DE)
(74) Vertreter: BASF IP Association
(86) Internationale Anmeldenummer: PCT/EP2020/067683
(87) Internationale Veröffentlichungsnummer: WO 2021/001240

(56) Entgegenhaltungen:
- WO-A1-2013/023307
- WO-A2-2012/052996
- ALESSANDRO DEL ZOTTO ET AL: "[RuCl 2 (PPh 3 )(PNN')] Complexes as Efficient Catalysts in Transfer Hydrogenation of Ketones", ORGANOMETALLICS, Bd. 26, Nr. 23, 1. November 2007 (2007-11-01), Seiten 5636-5642, XP055726810, ISSN: 0276-7333, DOI: 10.1021/om700647k
- Jing Zhang ET AL: "Efficient Homogeneous Catalytic Hydrogenation of Esters to Alcohols", Angewandte Chemie International Edition, vol. 45, no. 7, 6 February 2006 (2006-02-06), pages 1113-1115, XP055016953, ISSN: 1433-7851, DOI: 10.1002/anie.200503771

## Beschreibung

Die vorliegende Erfindung betrifft ein Verfahren zur Hydrierung eines Esters mit molekularem Wasserstoff zu den korrespondierenden Alkoholen in Gegenwart eines Ruthenium-Komplexes mit einem dreizähnigen PNN-Liganden.

Alkohole sind nicht nur wichtige Lösungsmittel, sondern auch wichtige Zwischenprodukte und Synthesebausteine, etwa zur Herstellung von Arznei- und Pflanzenschutzmitteln oder Riechstoffen. In Abhängigkeit von der Art des gewünschten Alkohols und der Verfügbarkeit des entsprechenden Ausgangsstoffes sind oftmals die direkte Hydrierung des entsprechenden Esters mit Wasserstoff oder auch die Reduktion mit Reduktionsmitteln die Methoden der Wahl.

Die Synthese von Alkoholen aus Estern erfolgt üblicherweise durch den Einsatz von Metallhydriden wie beispielsweise LiAlH₄ oder NaBH₄, durch heterogenkatalytische Hydrierung mit Wasserstoff, oder durch homogenkatalytische Hydrierung mit Wasserstoff. Homogenkatalytische Hydrierungen mit Wasserstoff ermöglichen oft weniger drastischen Reaktionsbedingungen bei gleichzeitig besserer Selektivität. Insbesondere der Einsatz von Ruthenium-Komplexen mit mehrzähnigen Phosphor-, Schwefel- und Stickstoff-haltigen Liganden hat sich gemäß dem Stand der Technik hierzu bewährt.

So beschreibt US 8,013,193 den Einsatz von Ru-Komplexen mit Triphos-Liganden wie beispielsweise 1,1,1-Tris(diphenylphosphinomethyl)ethan in der Hydrierung von Lactonen und Estern. Die genannten Komplexe zeigen allerdings eine eher niedrige Reaktionsgeschwindigkeit. Erst durch den Einsatz sehr spezieller aktivierender Lösungsmittel (2,2,2-Trifluorethanol) kann eine akzeptable Reaktionsgeschwindigkeit erreicht werden. Die zugeführten Lösungsmittel sind anschließend jedoch wieder vom Produkt abzutrennen.

Ein weiterer Nachteil der Triphos-Liganden ist das hohe Phosphor/Ruthenium-Molverhältnis von drei. Phosphin-Liganden sind in der Herstellung aufwändig. Zudem führen die drei Phosphino-Gruppen zu einer relativ hohen Molmasse des Komplexliganden sowie des einzusetzenden Ru-Komplexes. Hohe Molmassen sind in der allgemeinen Handhabung grundsätzlich nachteilig. Zudem ist der Aufwand bei der anschließenden Entsorgung des gebrauchten Ru-Komplexes aufwändiger, da mehr Masse zu entsorgen ist und zudem Phosphor-haltige Komponenten eine spezielle Entsorgung erfordern.

In einer Reihe verschiedener Publikationen sind Ru-Komplexe mit tetradentaten PNNP-Liganden zur Hydrierung von Estern zu Alkoholen beschrieben. So offenbaren Saudan et al. in Angewandte Chemie International Edition 2007, Vol. 46, Seiten 7473-7476, US 7,989,665, US 8,124,816, US 8,524,953 und US 9,193,651 unter anderem den Einsatz von Liganden des Typs 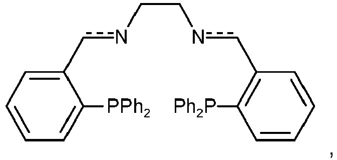 wobei die gestrichelte Linie jeweils für eine optionale Doppelbindung steht. Als weitere Liganden im sechsfach koordinierten Ru-Komplex sind insbesondere CI, H, BH₄, CO, OH, Alkoxy, Carboxy und Monophosphin genannt. Basierend auf einer Versuchsreihe von Ru-Komplexen mit di- und tetradentaten P- und N-haltigen Liganden weisen Saudan et al. in dem genannten Artikel darauf hin, dass für die Hydrierung von Estern zu Alkoholen zwei amino-phosphino-überbrückte Liganden im Ru-Komplex erforderlich sind. Diese können in Form eines PNNP-Liganden oder zweier PN-Liganden im Ru-Komplex vorliegen.

Nachteilig am Einsatz eines Ru-Komplexes mit einem PNNP-Liganden oder zweier PN-Liganden ist das hohe Phosphor/Ruthenium-Molverhältnis von zwei. Wie bereits zuvor beschrieben, sind Phosphin-Liganden in der Herstellung aufwändig. Zudem führen sie zu einer relativ hohen Molmasse des Ru-Komplexes, die durch ihre dann geringere Atomökonomie (mehr Masse pro Katalysatorkomplex) gegenüber kleineren Katalysatorkomplexen mit der gleichen katalytischen Aktivität von Nachteil ist.

Die Nachteile eines hohen Phosphor-Gehalts zeigt auch der in US 8,471,048 beschriebene tridentate PNP-Ligand der allgemeinen Struktur 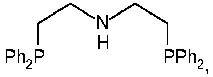 der ebenfalls für die Ru-katalysierte Hydrierung von Estern sowie von Ketonen und Lactonen zu Alkoholen vorbeschrieben ist.

Nachteilig an diesem Liganden ist die aufwändige Synthese über Bis(2-chlorethyl)-ammoniumchlorid und dessen Umsetzung mit Diphenylphosphin und Kalium-tert-butylat und nachfolgender Aufarbeitung mit HCl (siehe Whitesides et al. in J. Org. Chem. 1981, Vol. 46, Seiten 2861-2867).

Milstein et al. beschreiben in Angewandte Chemie International Edition 2006, Vol. 45, Seiten 1113-1115 den Einsatz von Ru-Komplexen mit einem sogenannten Pincer-Liganden vom Typ PNN zur Hydrierung von Estern zu Alkoholen. Der beschriebene tridentate Pincer-Ligand weist eine Pyridyl-Gruppe als Rückgrat und als Donor-Gruppen eine Phosphino- und Amino-Gruppe mit jeweils niedermolekularen Alkylgruppen auf. Namentlich genannt ist dabei der Einsatz von (2-(Di-tert-butylphosphinomethyl)-6-(diethylaminomethyl)pyridin 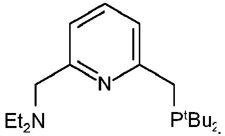 Ähnliche Pincer-Liganden vom Typ PNN sind auch in US 8,178,723 und US 2017/0,283,447 für die Hydrierung von Estern zu Alkoholen beschrieben.

Nachteilig am Einsatz der genannten Pincer-Liganden vom Typ PNN ist deren aufwändige, mehrstufige Synthese unter Verwendung anspruchsvoller Reagenzien ausgehend von 2,6-Dimethylpyridin, dessen Umsetzung mit N-Bromsuccinimid und Diethylamin zum 2-Diethylaminomethyl-6-methyl-pyridin, dessen nachfolgende Aktivierung der Methylgruppe durch Umsetzung mit Butyllithium und die abschließende Addition der P^{t}Bu₂-Gruppe durch Umsetzung mit Di-tert-butylphosphin.

US 2014/0,328,748 lehrt die Ru-katalysierte Hydrierung von Estern und Lactonen zu Alkoholen, katalysiert durch einen Ru-Komplex mit PNN-Ligand, welcher durch einen stickstoffhaltigen Heterozyklus, einer aliphatischen Brücke zu einem Aminstickstoff und von diesem durch eine weitere aliphatischen Brücke mit einer Länge von mindestens zwei Kohlenstoffen zu einer Phosphino-Gruppe gekennzeichnet ist. Als typische Vertreter dieser Kategorie sind PNN-Liganden der Struktur 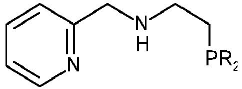 genannt, wobei R für einen Alkylrest wie etwa Isopropyl oder tert-Butyl oder einem Phenylrest steht.

Auch dieser Ligand ist aufgrund der -NH-CH₂CH₂-PR₂ Einheit nur durch eine aufwändige Synthese zugänglich. So erfolgt die Herstellung des Synthesebausteins H₂N-CH₂CH₂-PR₂ üblicherweise durch Umsetzung von HPR₂ mit dem aufwändig erhältlichen 2-Chlorethylamin und HCl. Des Weiteren sind Phosphine mit der Struktureinheit -CH₂CH₂-PR₂ (mit R = iPr) relativ oxidationsempfindlich, was sich durch eine geringe Lagerstabilität des Liganden beziehungsweise durch einen erhöhten Aufwand in der Handhabung aufgrund des Einsatzes einer Schutzgasatmosphäre negativ auswirkt.

WO 2012/052,996 lehrt die Hydrierung von Estern zu Alkoholen in Gegenwart eines Ru-Komplexes, welcher einen dreizähnigen PNN-Liganden der allgemeinen Struktur 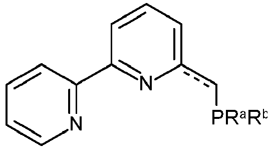 enthält, wobei die durchgezogen-gestrichelte Linie eine Einfach- oder Doppelbindung darstellt, die beiden aromatischen Systeme auch substituiert sein könne, und L unter anderem für Phosphin (PR^{a}R^{b} mit R^{a}, R^{b} gleich aromatischem oder nicht-aromatischem Rest) steht.

Zhang et al. offenbaren in Chemistry An Asian Journal 2016, Vol. 11, Seiten 2103-2106 den Einsatz eines Ru-Komplexes mit einem tetradentaten Bipyridin-Liganden als Katalysator für die Hydrierung von Estern zu Alkoholen. Konkret geht es darin um die beiden PNNN-Liganden

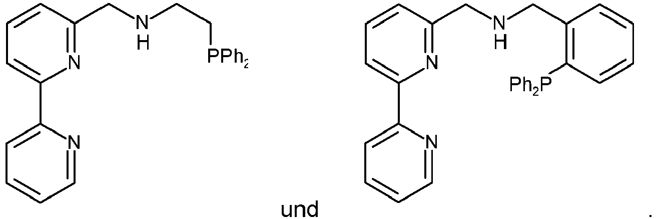

Die Autoren lehren dabei, dass das Bipyridin-Fragment essentiell für eine hohe Katalysatoraktivität sowie relevant zur Erreichung einer hohen Acidität der NH-Gruppe ist.

Auch diese Liganden weisen signifikante Nachteile auf. So ist deren Synthese aufgrund des Bipyridin-Fragments aus den gängigen Synthesebausteinen aufwändig. Ausgehend von 2-Brompyridin und 2-(Diphenylphosphaneyl)ethylamin beziehungsweise (2-(Diphenylphosphaneyl)phenyl)methylamin bedarf es jeweils einer vierstufigen Synthese unter Einsatz von n-Butyllithium. Der oben dargestellte Ligand mit der -CH₂C₆H₄-PPh₂ Einheit weist zusätzlich eine relativ hohe Molmasse auf. Die negativen Auswirkungen einer höheren Molmasse sind weiter oben bereits genannt. Des Weiteren zeigen die katalytischen Untersuchungen in der zitierten Literaturstelle, dass der Ligand mit der -CH₂C₆H₄-PPh₂-Einheit Ausbeuten von > 60% nur in Toluol als Lösungsmittel und auch in Gegenwart von Natriumalkoxiden als Base ermöglicht.

Rigo et al. lehren in Organometallics 2007, Vol. 26, Seiten 5636-5642 den Einsatz von tridentaten PNN-Liganden der Strukturen 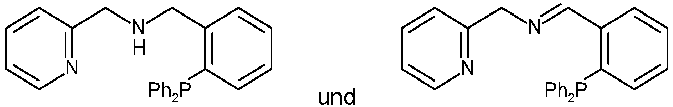 nach deren Umsetzung mit RuCl₂(PPh₃)₃ zum entsprechenden Ru-Komplex in der Transferhydrierung von Ketonen mit 2-Propanol zum entsprechenden sekundären Alkohol und Aceton. Bei der Transferhydrierung wird somit als reduzierendes Reagenz nicht Wasserstoff, sondern eine reduzierende Verbindung wie beispielsweise ein sekundärer Alkohol oder HCOOH/Amin eingesetzt. Es ist jedoch bekannt, dass Katalysatoren, welche für die Transferhydrierung von Ketonen zu Alkoholen gut geeignet sind, üblicherweise in der Hydrierung von Estern zu Alkoholen nicht ausreichend aktiv sind.

Transferhydrierkatalysatoren für Ketone unterscheiden sich strukturell und von Ihrer Reaktivität her von Hydrierkatalysatoren für Ester.

So zeigen beispielsweise Noyori et al. in Journal of the American Chemical Society 1996, Vol. 118, Seiten 2521-2522, dass ein zur Transferhydrierung von Ketonen zu Alkoholen sehr gut geeigneter Ru-Komplex, konkret (R)-RuCl[(1S,2S)-p-TsNCH(C₆H₅)CH-(C₆H₅)NH₂](η-Mesitylen), in der Transferhydrierung von Ketonen ohne Mühe Ausbeuten von 99% erreicht, in der Hydrierung mit Wasserstoff jedoch nur eine Ausbeute von 5% ermöglicht. Des Weiteren zeigen Noyori et al. auch, dass bei Substraten, die neben einer Keto-Funktion auch noch eine Ester-Funktion aufweisen, bei der Transferhydrierung nur die Keto-Funktion, nicht aber die Ester-Funktion zum entsprechenden Alkohol reduziert wird.

WO 2017/134,618 offenbart den Einsatz von Monocarbonylkomplexen des Rutheniums und Osmiums, welche des Weiteren einen Stickstoff- und Phosphorhaltigen Liganden enthalten in der Transferhydrierung von Ketonen und Aldehyden sowie der Hydrierung von Ketonen und Aldehyden mit Wasserstoff zu den korrespondierenden Alkoholen. Unter der Vielzahl der erwähnten Stickstoff- und Phosphorhaltigen Liganden ist unter anderem auch ein tridentater PNN-Ligand der Struktur 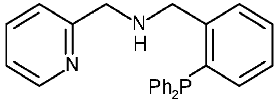 offenbart. WO 2017/134,618 bestätigt über viele Beispiele, dass Ru-Komplexkatalysatoren, die in der Transferhydrierung von Ketonen hohe Ausbeuten erreichen, in der Hydrierung mit Wasserstoff signifikant schlechtere Umsätze aufweisen. So zeigen beispielsweise die Ru-Komplexkatalysatoren mit den Nummern 16, 20 und 22 in der Transferhydrierung von Acetophenon mit 2-Propanol zum 1-Phenylethanol jeweils 100% Umsatz, wohingegen dieselben Ru-Komplexkatalysatoren in der Hydrierung von Acetophenon mit Wasserstoff nur Umsätze von 25 bis 63% ermöglichen. Der Ru-Komplexkatalysator mit dem oben genannten PNN-Liganden ([Ru(OAc)₂(CO)(PNN)] mit der Nummer 39) erreichte in der Transferhydrierung von Acetophenon mit 2-Propanol zum 1-Phenylethanol lediglich 96% Umsatz und wurde in der Hydrierung von Acetophenon mit Wasserstoff nicht getestet.

Zusammenfassend kann festgehalten werden, dass die im Stand der Technik für die homogenkatalysierte Hydrierung von Estern zu Alkoholen beschriebenen Liganden relativ hohe Molmassen besitzen, und/oder relativ aufwändig und umständlich herzustellen sind, und/oder nur eine eher niedrige chemische Stabilität aufweisen. Zudem ist allgemein bekannt, dass Liganden, welche in der Transferhydrierung von Ketonen zu Alkoholen zu sehr guten Resultaten führen, üblicherweise in der Hydrierung mit Wasserstoff als Reduktionsmittel weniger gut geeignet sind und insbesondere in der Hydrierung von Estern in den meisten Fällen ungeeignet sind.

Aufgabe der vorliegenden Erfindung war es, ein Verfahren zur homogenkatalysierten Hydrierung von Estern zu den korrespondierenden Alkoholen zu finden, welches die genannten Nachteile des Standes der Technik nicht oder in nur untergeordnetem Maße aufweist, hinsichtlich der erforderlichen Apparate und Reaktionsbedingungen einfach durchzuführen ist und eine möglichst hohe Raum-Zeit-Ausbeute ermöglicht.

Insbesondere soll der katalytisch aktive Komplex direkt aus gut verfügbaren Einsatzstoffen herstellbar sein, eine hohe Aktivität in der Hydrierung von Estern zu Alkoholen aufweisen und letztendlich auch ohne übermäßig hohen Aufwand zu entsorgen sein. In diesem Zusammenhang kommt vor allem dem komplexbildenden Liganden eine besondere Bedeutung zu. Um insgesamt mit möglichst wenig Katalysatormasse auszukommen, hat ein bevorzugter Ligand bei vergleichbarer Katalysatoraktivität und auch vergleichbaren Aufwand in der Herstellung eine möglichst niedrige Molmasse aufzuweisen. Zudem ist eine hohe chemische Stabilität des Liganden wünschenswert, so dass dieser vor dessen Einsatz ohne besonders aufwändige Maßnahmen lagerstabil ist und auch während des Einsatzes stabil bleibt.

Zudem sollte der katalytisch aktive Komplex zur Hydrierung einer Vielzahl von Estern unabhängig von deren Molmasse und weiteren Struktur einsetzbar sein.

Überraschend wurde ein Verfahren zur Hydrierung eines Esters mit molekularem Wasserstoff zu den korrespondierenden Alkoholen bei einer Temperatur von 50 bis 200°C und einem Druck von 0,1 bis 20 MPa abs in Gegenwart eines fünffach oder sechsfach koordinierten Ruthenium-Komplexes (I), wobei der Ruthenium-Komplex auch zu einem Dimer verbrückt sein kann, gefunden, bei dem der Ruthenium-Komplex einen dreizähnigen Liganden L mit der allgemeinen Formel (II) 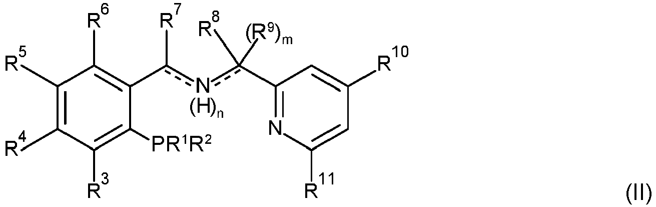 enthält, wobei
R¹, R² unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
R³, R⁴, R⁵, R⁶, R¹⁰, R¹¹ unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
R⁷, R⁸, R⁹ unabhängig voneinander für Wasserstoff, lineares C₁ bis C₄-Alkyl oder verzweigtes C₃ bis C₄-Alkyl stehen,
n, m unabhängig voneinander 0 oder 1 sind, und
die durchgezogen-gestrichelten Doppellinien für eine Einfach- oder Doppelbindung stehen, mit der Maßgabe, dass

| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung darstellt, wobei im Falle einer Doppelbindung auf der dem Phenylring zugewandten Seite m = 1 ist, im Falle einer Doppelbindung auf der dem Pyridylring zugewandten Seite m = 0 ist, |
| | oder beide durchgezogen-gestrichelten Doppellinien eine Einfachbindung darstellen und m gleich 1 ist. |

Kern des erfindungsgemäßen Verfahrens ist der Einsatz eines fünffach oder sechsfach koordinierten Ruthenium-Komplexes (I), welcher einen dreizähnigen Liganden L der allgemeinen Formel (II) enthält, in der Hydrierung von Estern mit molekularem Wasserstoff zu den korrespondierenden Alkoholen.

Bei dem dreizähnigen Liganden L handelt es sich um einen sogenannten PNN-Liganden mit der allgemeinen Formel (II) 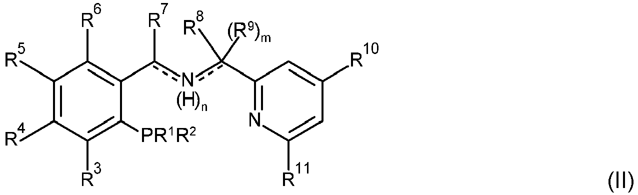 in der
- R¹, R²: unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
- R³, R⁴, R⁵, R⁶, R¹⁰, R¹¹: unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
- R⁷, R⁸, R⁹: unabhängig voneinander für Wasserstoff, lineares C₁ bis C₄-Alkyl oder verzweigtes C₃ bis C₄-Alkyl stehen, n, m unabhängig voneinander 0 oder 1 sind, und
die durchgezogen-gestrichelten Doppellinien für eine Einfach- oder Doppelbindung stehen, mit der Maßgabe, dass

| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung darstellt, wobei im Falle einer Doppelbindung auf der dem Phenylring zugewandten Seite m = 1 ist, im Falle einer Doppelbindung auf der dem Pyridylring zugewandten Seite m = 0 ist, oder beide durchgezogen-gestrichelten Doppellinien eine Einfachbindung darstellen und m = 1 ist. |

Dreizähnig bedeutet, dass Ligand L (II) drei Koordinationsstellen im Ruthenium-Komplex (I) besetzt. Bei den drei Liganden-Donoratomen handelt es sich um das P und die beiden N-Atome, woraus sich auch die Bezeichnung PNN-Ligand ableitet.

Hinsichtlich der Umgebung des mittleren Donoratoms kann der Ligand grundsätzlich vier verschiedene Substrukturen aufweisen, die im Folgenden näher erläutert sind.
(1) Im Falle von n = 1 stellen beide durchgezogen-gestrichelte Doppellinien eine Einfachbindung dar und m beträgt 1. Daraus resultiert die allgemeine Formel (IIa). Ligand (IIa) ist neutral, besitzt also die Ladung "0". 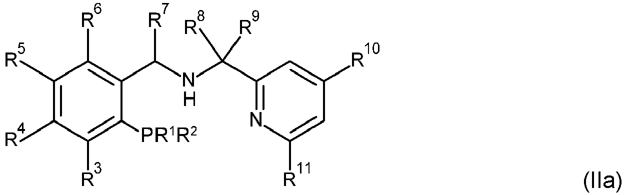 Im Falle von n = 0 gibt es insgesamt drei verschiedene Substrukturen.
(2) Ist n = 0 und die dem Phenylring zugewandte durchgezogen-gestrichelte Doppellinie eine Doppelbindung und die dem Pyridylring zugewandte durchgezogen-gestrichelte Doppellinie eine Einfachbindung, so ist m gleich 1. Daraus resultiert die allgemeine Formel (IIb). Ligand (IIb) ist neutral, besitzt also die Ladung "0". 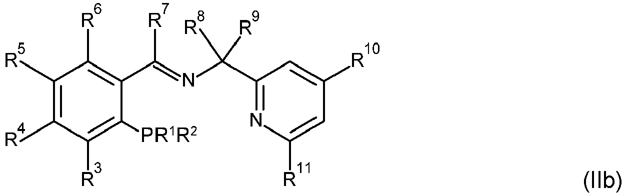
(3) Ist n = 0 und die dem Pyridylring zugewandte durchgezogen-gestrichelte Doppellinie eine Doppelbindung und die dem zugewandte Phenylring durchgezogen-gestrichelte Doppellinie eine Einfachbindung, so ist m gleich 0. Daraus resultiert die allgemeine Formel (IIc). Ligand (IIc) ist neutral, besitzt also die Ladung "0". 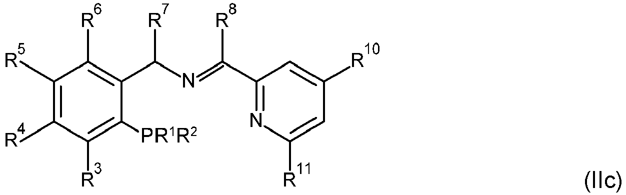
(4) Bei der vierten Variante ist ebenfalls n = 0, jedoch sind beide durchgezogen-gestrichelten Doppellinien Einfachbindungen und m ist 1. Das N-Atom weißt somit eine negative Ladung auf. Daraus resultiert die allgemeine Formel (IId). Der Ligand (IId) besitzt somit die Ladung "-1".

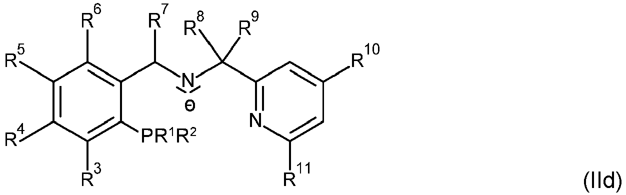

Die Reste R¹ und R² des Liganden (II) können in einem weiten Umfang variieren und stehen unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen, wobei die genannten Kohlenwasserstoffreste unsubstituiert oder mit 1 bis 3 Methoxy-, Thiomethoxy- oder Dimethylamino-Gruppen substituiert sein können, und die beiden Reste R¹ und R² unter Bildung eines, das Phosphoratom einschließenden 5 bis 10-Ringes auch miteinander verbunden sein können.

Im Fall eines aliphatischen Kohlenwasserstoffrests kann dieser unverzweigt oder verzweigt beziehungsweise linear oder cyclisch sein. Bevorzugt besitzen die aliphatischen Kohlenwasserstoffrest 1 bis 6 Kohlenstoffatome, besonders bevorzugt 1 bis 4 Kohlenstoffatome und ganz besonders bevorzugt 1 bis 2 Kohlenstoffatome. Als konkrete Beispiele seien genannt Methyl, Ethyl, iso-Propyl, n-Propyl, n-Butyl, iso-Butyl, tert.-Butyl (auch als tBu bezeichnet) und Cyclohexyl (auch als Cy bezeichnet).

Im Fall eines aromatischen Kohlenwasserstoffrests handelt es sich um Phenyl (auch als Ph bezeichnet), 1-Naphthyl oder 2-Naphthyl.

Araliphatischen Kohlenwasserstoffreste enthalten aromatische und aliphatische Elemente, unabhängig davon, ob diese über eine aliphatische oder eine aromatische Gruppe an das Phosphoratom im Liganden L gebunden sind. Bevorzugt besitzen die araliphatischen Kohlenwasserstoffreste 7 bis 10 Kohlenstoffatome und besonders bevorzugt 7 bis 9 Kohlenstoffatome. Als konkrete Beispiele seien genannt o-Tolyl, m-Tolyl, p-Tolyl und Benzyl.

Im Fall eines, das Phosphoratom einschließenden Ringes, handelt es sich bevorzugt um einen Ring mit 5 bis 6 Atomen, einschließlich des Phosphoratoms. Als Beispiele seien genannt Butan-1,4-diyl, Pentan-1,5-diyl und 2,4-Dimethylpentan-1,5-diyl.

Die genannten aliphatischen, aromatischen und araliphatischen Kohlenwasserstoffreste, die auch unter Bildung eines, das Phosphoratom einschließenden Ringes miteinander verbunden sein können, können unsubstituiert oder mit 1 bis 3 Methoxy-, Thiomethoxy- oder Dimethylamino-Gruppen substituiert sein. Die oben genannte Anzahl der Kohlenstoffatome der einzelnen Kohlenwasserstoffreste ist einschließlich der Kohlenstoffatome der Methoxy-, Thiomethoxy- beziehungsweise Dimethylamino-Gruppen zu verstehen. Als konkrete Beispiele seien genannt 3,5-Dimethyl-phenyl, 3,5-Dimethyl-4-methoxy-phenyl, 3,5-Dimethyl-4-thiomethoxy-phenyl und 3,5-Dimethyl-4-(dimethylamino)phenyl.

Besonders bevorzugt handelt es sich bei den Resten R¹ und R² Phenyl, p-Tolyl, o-Tolyl, 4-Methoxyphenyl, 2-Methoxyphenyl, Cyclohexyl, iso-Butyl, tert-Butyl, 3,5-Dimethyl-4-methoxyphenyl, 3,5-tert-Butyl-4-methoxyphenyl und 3,5-Dimethyl-phenyl und ganz besonders bevorzugt um Phenyl, p-Tolyl, 3,5-Dimethyl-4-methoxyphenyl, iso-Butyl und Cyclohexyl, wobei bevorzugt jeweils beide Reste gleich sind.

Die Reste R³, R⁴, R⁵, R⁶, R¹⁰ und R¹¹ stehen unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe. Als lineares C₁ bis C₄-Alkyl sind Methyl, Ethyl, n-Propyl und n-Butyl sowie als verzweigtes C₃ bis C₄-Alkyl iso-Propyl, sec.-Butyl und tert.-Butyl zu nennen. Als Dialkylamino sind insbesondere Amino-Reste mit identischen Alkylgruppen zu nennen, insbesondere Dimethylamino, Diethylamino, Di-n-propylamino und Di-n-butylamino.

Bevorzugt stehen die Reste R³ und R⁴ unabhängig voneinander für Wasserstoff oder Methyl und besonders bevorzugt für Wasserstoff.

Bevorzugt steht der Rest R⁵ für Wasserstoff, Methyl, iso-Propyl, sec.-Butyl, tert.-Butyl, Methoxy, Hydroxy oder Dialkylamino, besonders bevorzugt für Wasserstoff, Methyl oder Hydroxy und ganz besonders bevorzugt für Wasserstoff.

Bevorzugt steht der Rest R⁶ für Wasserstoff.

Bevorzugt steht der Rest R¹⁰ für Wasserstoff, Methyl, iso-Propyl, sec.-Butyl, tert.-Butyl oder Methoxy, besonders bevorzugt für Wasserstoff, Methyl oder tert.-Butyl und ganz besonders bevorzugt für Wasserstoff.

Bevorzugt steht der Rest R¹¹ für Wasserstoff, Methyl, Ethyl, Methoxy, Ethoxy oder iso-Propyloxy und besonders bevorzugt für Wasserstoff, Methyl oder Methoxy.

Ganz besonders bevorzugt sind Liganden (II), bei denen
- R³, R⁴, R⁵, R⁶, R¹⁰ und R¹¹ für Wasserstoff stehen,
- R³, R⁴, R⁵, R⁶ und R¹⁰ für Wasserstoff und R¹¹ für Methyl stehen,
- R³, R⁴, R⁵, R⁶ und R¹⁰ für Wasserstoff und R¹¹ für Methoxy stehen,
- R³, R⁴, R⁶, R¹⁰ und R¹¹ für Wasserstoff und R⁵ für Methyl stehen,
- R³, R⁴, R⁶, R¹⁰ und R¹¹ für Wasserstoff und R⁵ für tert.-Butyl stehen
- R³, R⁴, R⁵, R⁶ und R¹¹ für Wasserstoff und R¹⁰ für Methyl stehen,
- R³, R⁴, R⁵, R⁶ und R¹¹ für Wasserstoff und R¹⁰ für tert.-Butyl stehen, sowie
- R³, R⁴, R⁵ und R⁶ für Wasserstoff und R¹⁰ und R¹¹ für Methyl stehen.

Die Reste R⁷, R⁸ und R⁹ stehen unabhängig voneinander für Wasserstoff, lineares C₁ bis C₄-Alkyl oder verzweigtes C₃ bis C₄-Alkyl. Als lineares C₁ bis C₄-Alkyl sind Methyl, Ethyl, n-Propyl und n-Butyl sowie als verzweigtes C₃ bis C₄-Alkyl iso-Propyl, sec.-Butyl und tert.-Butyl zu nennen.

Bevorzugt stehen die Reste R⁷, R⁸ und R⁹ unabhängig voneinander für Wasserstoff, Methyl, Ethyl oder n-Propyl, besonders bevorzugt für Wasserstoff oder Methyl und ganz besonders bevorzugt für Wasserstoff.

Ganz besonders bevorzugt sind Liganden (II), bei denen
- R⁷, R⁸ und R⁹ für Wasserstoff stehen,
- R⁷ und R⁹ für Wasserstoff und R⁸ für Methyl stehen,
- R⁷ für Wasserstoff und R⁸ und R⁹ für Methyl stehen,
- R⁷ für Methyl und R⁸ und R⁹ für Wasserstoff stehen, und
- R⁷ und R⁸ für Methyl und R⁹ für Wasserstoff stehen.

Besonders vorteilhaft beim erfindungsmäßen Verfahren ist der Einsatz der Liganden (II), bei denen
(i) n und m jeweils 1 ist und die beiden durchgezogen-gestrichelten Doppellinien eine Einfachbindung darstellen (Struktur (IIa)), oder
(ii) n gleich 0 und m gleich 1 ist und die dem Phenylring zugewandte durchgezogen-gestrichelte Doppellinie eine Doppelbindung und die dem Pyridylring zugewandte durchgezogen-gestrichelte Doppellinie eine Einfachbindung darstellt (Struktur (IIb)),
   und

- beide Reste R¹ und R² für Phenyl, p-Tolyl, 3,5-Dimethyl-4-methoxyphenyl, iso-Butyl oder Cyclohexyl stehen,
- die Reste R³, R⁴ und R⁶ für Wasserstoff stehen,
- die Reste R⁵ und R¹⁰ für Wasserstoff, Methyl oder tert.-Butyl stehen,
- der Rest R¹¹ für Wasserstoff, Methyl oder Methoxy steht, und
- die Reste R⁷, R⁸ und R⁹ für Wasserstoff oder Methyl stehen.

Ligand (II) kann auf einfache Art und Weise durch Kondensation eines entsprechenden Amins mit einem entsprechenden Aldehyd oder Keton (Ligand (IIb) und (Ilc)) sowie einer eventuell nachfolgenden Reduktion (Ligand (IIa)) sowie einer eventuell nachfolgenden Deprotonierung unter basischen Bedingungen (Ligand (!!d)) erhalten werden.

Bei der Kondensation gibt es grundsätzlich zwei verschiedene Möglichkeiten. Zum einen ist es möglich, als Aminkomponente 2-Picolylamin oder ein entsprechendes Derivat davon, und als Aldehyd- oder Ketonkomponente einen entsprechend substituierten Phosphanylbenzaldehyd oder ein entsprechendes Keton einzusetzen.

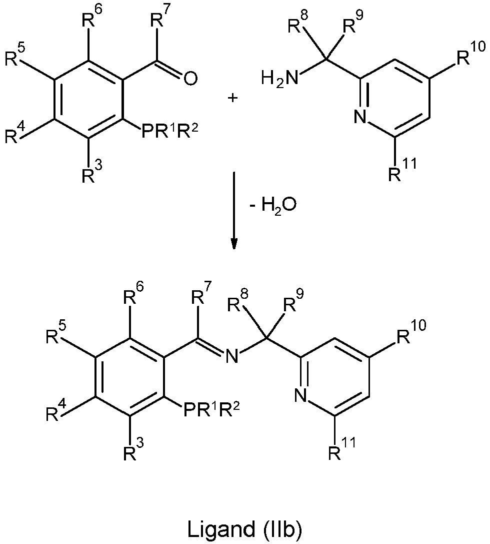

Zum anderen ist es aber auch möglich, als Aminkomponente ein entsprechend substituiertes Phosphanylphenylmethanamin und als Aldehyd- oder Ketonkomponente Picolinaldehyd oder ein entsprechendes Derivat davon einzusetzen.

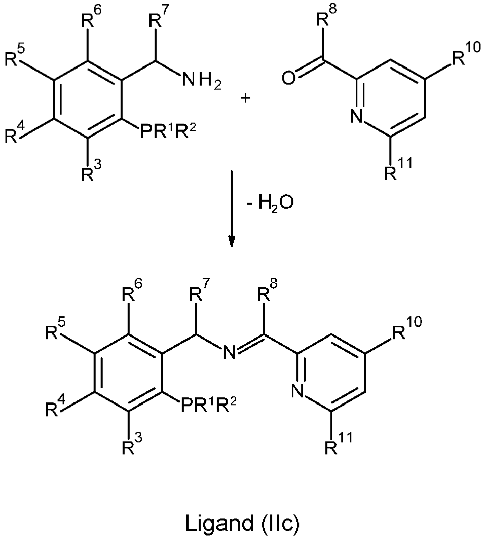

Die entsprechenden Ausgangsverbindungen (Amine, Ketone oder Aldehyde) sind in der Regel kommerziell erhältlich oder können mit allgemein bekannten Methoden synthetisiert werden. Die Synthese der Liganden (Ilb) und (IIc) erfolgt üblicherweise unter Schutzgasatmosphäre. Die beiden Komponenten werden dabei üblicherweise in einem Lösungsmittel bei einer Temperatur von 50 bis 200°C miteinander umgesetzt. Als geeignete Lösungsmittel seien beispielhaft aliphatische Alkohole wie beispielsweise Methanol, Ethanol oder Isopropanol sowie aromatische Kohlenwasserstoffe wie etwa Toluol oder Xylole genannt. Die beiden Ausgangsverbindungen können in stöchiometrischer Menge eingesetzt werden. Es ist aber auch möglich, eine der beiden Komponenten im Überschuss einzusetzen, um beispielsweise den Umsatz der anderen Komponente zu erhöhen. Dies ist insbesondere sinnvoll, wenn die andere Komponente schwer zugänglich ist. Wird ein Überschuss eingesetzt, so liegt das Molverhältnis der beiden Ausgangsverbindungen in der Regel im Bereich von > 1 bis ≤ 2. Als Reaktionszeit reichen üblicherweise wenige Minuten bis mehrere Stunden. Als typische Reaktionszeit seien 10 Minuten bis 5 Stunden und bevorzugt 30 Minuten bis 3 Stunden genannt. Die Aufarbeitung des Reaktionsgemisches und Isolierung des Liganden können nach üblichen Methoden erfolgen. Vorteilhafterweise entfernt man jedoch das zugesetzte Lösungsmittel und Wasser im Vakuum.

Die Liganden (IIb) und (IIc) können nun zur Darstellung des Ruthenium-Komplexes (I) eingesetzt werden.

Eine besonders elegante und daher auch bevorzugte Variante ist die Synthese der Liganden (IIb) und (IIc) zusammen mit der Darstellung des Ruthenium-Komplexes (I) in einer Eintopfreaktion. Hierzu werden die Ausgangsverbindungen (Amine und Keton oder Aldehyd) zunächst wie oben beschrieben miteinander umgesetzt, jedoch anschließend nicht aufgearbeitet und isoliert, sondern zum erhaltenen Reaktionsgemisch der entsprechende Ruthenium-Precursor sowie gegebenenfalls eine Base zur Bildung des katalytisch aktiven Ruthenium-Komplexes (I) hinzugefügt. Unter Einstellung der Hydrierbedingungen und Zugabe des zu hydrierenden Esters ist es somit sogar sehr einfach möglich, in einer Eintopfreaktion den Liganden (II) herzustellen, daraus im direkten Anschluss den Ruthenium-Komplexes (I) darzustellen und im direkten Anschluss auch die Hydrierung des Esters durchzuführen.

Durch Reduktion von Ligand (IIb) oder (IIc) mit Reduktionsmitteln wie beispielsweise Natriumborhydrid oder Lithiumaluminiumhydrid oder katalytisch mit Wasserstoff kann aus den Liganden (Ilb) und (IIc) in einfacher Art und Weise der Ligand (IIa) gewonnen werden. Die Umsetzung kann mit dem üblichen Wissen des Fachmanns durchgeführt werden.

In einer besonders vorteilhaften Synthese führt man die oben beschriebene Kondensation und die Reduktion zum Liganden (IIa) in einer Eintopfreaktion direkt hintereinander durch, ohne die Liganden (IIb) und (IIc) vorab zwischen zu isolieren. Hierzu gibt man nach dem Ablauf der Kondensation das Reduktionsmittel direkt zum Reaktionsgemisch und lässt es für eine weitere Zeit miteinander reagieren. Auch hierzu reichen üblicherweise wenige Minuten bis mehrere Stunden. Als typische Reaktionszeit seien 10 Minuten bis 5 Stunden und bevorzugt 30 Minuten bis 3 Stunden genannt. Anschließend kann die Aufarbeitung des Reaktionsgemisches und Isolierung des Liganden nach üblichen Methoden erfolgen. Auf die zur Aufarbeitung und Isolierung der Liganden (IIb) und (IIc) genannten Angaben sei explizit hingewiesen.

Ligand (IIa) bildet sich aus den Liganden (IIb) und (IIc) auch gebunden im Ruthenium-Komplex (I) unter Reaktionsbedingungen durch Hydrierung mit dem zugeführten Wasserstoff.

Der anionische Ligand (IId) bildet sich aus Ligand (IIa) durch Umsetzung mit einer starken Base infolge einer Abspaltung des am Stickstoff befindlichen Wasserstoffatoms als Proton. Geeignete starke Basen sind beispielsweise NaOMe oder KOMe. Üblicherweise führt man diese Umsetzung nicht gezielt mit dem freien Liganden (Ila) durch.

Vielmehr kann sich der Ligand (IId) im Ruthenium-Komplex (I) unter Hydrierbedingungen in Gegenwart einer starken Base bilden.

Der beim erfindungsgemäßen Verfahren einzusetzende Ruthenium-Komplex (I) ist fünffach oder sechsfach koordiniert. Davon sind drei Koordinationsstellen bereits vom dreizähnigen Liganden (II) besetzt. Der Ruthenium-Komplex (I) kann dabei einkernig oder zweikernig, also auch als Dimer verbrückt, vorliegen. Ist der Ruthenium-Komplex (I) zu einem Dimer verbrückt, weist dieser zwei Ruthenium-Atome im Komplex auf.

Beim erfindungsgemäßen Verfahren ist die Oxidationsstufe des Rutheniums im Ruthenium-Komplex (I) nicht eingeschränkt. In der Regel beträgt diese jedoch 0 (null), +2 oder +3 und bevorzugt +2 oder +3.

Der beim erfindungsgemäßen Verfahren bevorzugt eingesetzte Ruthenium-Komplex (I) enthält Ruthenium in der Oxidationsstufe +2 oder +3 und besitzt die allgemeine Formel (IA)

[Ru(L)XₐY_{b}]ₚ Z_{(p · c)} (IA)

wobei
X jeweils unabhängig voneinander für einen neutralen monodentaten Liganden steht, wobei zwei Liganden X auch zu einem neutralen bidentaten Liganden verbunden sein können,
Y jeweils unabhängig voneinander für einen anionischen monodentaten Liganden mit der Ladung "-1" steht,
wobei Y und X zusammen auch für einen anionischen bidentaten Liganden mit der Ladung "-1" stehen kann,
Z jeweils unabhängig voneinander für ein nicht-koordinierendes Anion mit der Ladung "-1" steht, wobei zwei Liganden Z auch zu einem nicht-koordinierenden Anion mit der Ladung "-2" verbunden sein können,
a, b und c jeweils unabhängig voneinander für 0, 1, 2 oder 3 stehen, und
p für 1 oder 2 steht,
mit den Maßgaben, dass
a + b + c gleich 1, 2, 3, 4, 5 oder 6 ist, und
b und c derart bestimmt sind, dass der Ruthenium-Komplex (IA) die Gesamtladung "0" aufweist.

Im Ruthenium-Komplex (IA) geben die Indizes a, b und c jeweils die Anzahl der jeweiligen Liganden X, Y und des nicht koordinierenden Gegenions Z bezogen auf ein Ruthenium-Atom vor. Da Ligand (II) dreizähnig ist und der Ruthenium-Komplex (IA) maximal sechsfach koordiniert ist, liegt der maximale Wert der Indizes jeweils bei 3.

Der Index p gibt an, ob der Ruthenium-Komplex (IA) einkernig (p = 1) oder zweikernig (p = 2) ist.

Durch die Einhaltung weiterer Randbedingungen folgt, dass die Summe aus a, b und c nur die Werte 1, 2, 3, 4, 5 und 6 annehmen kann. Da der Ruthenium-Komplex (IA) insgesamt als neutral definiert ist, sind keine beliebigen Kombinationen für a, b und c möglich.

Der Vollständigkeit halber sei noch explizit darauf hingewiesen, dass der Index "(p · c)" in der allgemeine Formel (IA) für das Produkt aus "p mal c" steht.

Als Ruthenium-Komplex (IA) ist beim erfindungsgemäßen Verfahren ein Ruthenium-Komplex bevorzugt, bei dem
- X: jeweils unabhängig voneinander für einen neutralen Liganden ausgewählt aus der Gruppe CO, NH₃, NR₃, R₂NSO₂R, PR₃, AsR₃, SbR₃, P(OR)₃, SR₂, RCN, RNC, N₂, NO, PF₃, Pyridin, Thiophen, Tetrahydrothiophen und N-heterocyclisches Carben der allgemeinen Formeln 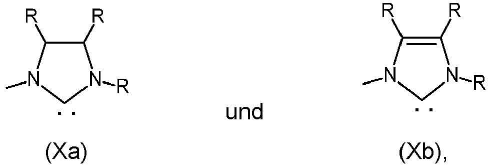 steht oder zwei Liganden X zusammen für 1,5-Cyclooctadien stehen,
- Y: jeweils unabhängig voneinander für einen anionischen Liganden ausgewählt aus der Gruppe H-, F-, CI-, Br-, I-, OH-, C₁ bis C₆-Alkoxy, C₁ bis C₆-Carboxy, Methylallyl, Acetylacetonato, RSO₃⁻, CF₃SO₃⁻, CN- und BH₄⁻ steht,
oder ein Y zusammen mit einem X für C₁ bis C₆-Carboxy oder Acetylacetonato steht; und
- Z: jeweils unabhängig voneinander für ein nicht-koordinierendes Anion ausgewählt aus der Gruppe H-, F-, CI-, Br, I⁻, OH-, BF₄⁻, PF₆⁻, NO₃⁻, RCOO⁻, CF₃COO⁻, CH₃SO₃⁻ , CF₃SO₃⁻, BH₄⁻, NH₂⁻, RO⁻, CN⁻, R₂N⁻, SCN⁻, OCN⁻, RS⁻, R-CONH⁻, (R-CO)₂N⁻, HCO₃⁻, HSO₄⁻, H₂PO₄⁻, Acetylacetonat, Pentafluorbenzoat, Bis(trimethylsilyl)amid und Tetrakis[3,5-bis(trifluoromethyl)phenyl]borat steht oder zwei Liganden Z zusammen für CO₃²⁻, SO₄²⁻, HPO₄²⁻, S²⁻ stehen,
wobei die Reste R in den Definitionen von X, Y und Z jeweils unabhängig voneinander C₁ bis C₁₀-Alkyl, CF₃, C₂F₅, C₃ bis C₁₀-Cycloalkyl, C₃ bis C₁₀-Heterocyclyl enthaltend mindestens ein Heteroatom ausgewählt aus der Gruppe N, O und S, C₅ bis C₁₀-Aryl oder C₅ bis C₁₀-Heteroaryl enthaltend mindestens ein Heteroatom ausgewählt aus der Gruppe N, O und S, bedeuten.

Bevorzugt stehen die Reste R unabhängig voneinander für C₁ bis C₄-Alkyl, C₅ bis C₆-Cycloalkyl, o-Tolyl, p-Tolyl, Xylyl oder Mesityl und besonders bevorzugt für Methyl, Xylyl oder Mesityl.

Bevorzugt steht der neutrale Ligand X im Ruthenium-Komplex (IA) für CO, Trimethylphosphin, Triphenylphosphin, Tricyclohexylphoshin, Triphenylphosphit oder Trimethylphosphit, oder zwei Liganden X zusammen für Cycloocta-1,5-dien. Besonders bevorzugt steht der neutrale Ligand X für Triphenylphosphin.

Bevorzugt steht der anionische Ligand Y im Ruthenium-Komplex (IA) für H⁻, Cl⁻, OH⁻, C₁ bis C₄-Alkoxy, C₁ bis C₄-Carboxy, Methylallyl, Acetylacetonato oder Bis(trimethylsilyl)amid, oder ein Ligand Y zusammen mit einem Liganden X für C₁ bis C₄-Carboxy oder Acetylacetonato. Besonders bevorzugt steht der anionische Ligand Y für H⁻, Cl⁻, Methoxy oder Acetat.

Anionische Liganden mit einer anionischen O- Gruppe und einer neutralen O= Gruppe, welche über eine ungerade Anzahl an Kohlenstoffatomen miteinander verbunden sind, wie beispielsweise Carboxylate oder Acetylacetonat, können sowohl als einzähnige als auch als zweizähnige Liganden fungieren, wie die nachfolgenden Beispiele mit dem

Acetatanion zeigen.

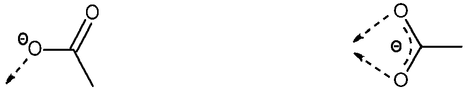

Falls der Komplex mit den Liganden L, X und Y eine positive Ladung aufweisen sollte, ist noch eine entsprechende Anzahl an nicht-koordinierenden Anionen Z erforderlich, um den Ruthenium-Komplex (IA) neutral zu stellen. Bevorzugt sind dabei Anionen, bei denen Z für Cl⁻, OH-, C₁ bis C₄-Alkoxylat, C₁ bis C₄-Carboxylat, BF₄⁻ oder PF₆⁻ steht, oder zwei Z zusammen für SO₄²⁻ stehen. Besonders bevorzugt steht das nicht-koordinierende Anionen Z für CI-, Methanolat, Acetat, BF₄⁻ oder PF₆⁻.

Besonders bevorzugt ist ein Verfahren, bei dem der Ruthenium-Komplex (IA) ausgewählt ist aus der Gruppe bestehend aus
(a) Ruthenium-Komplex (IAa) der allgemeinen Formel (IAa)

   [Ru(L)X₁₊ₚY₂₋ₚ] Zₚ (IAa)

   mit p = 0 oder 1;
(b) Ruthenium-Komplex (lAb) der allgemeinen Formel (lAb)

   [Ru(L)XₚY₂₋ₚ] Zₚ (lAb)

   mit p = 0 oder 1;
(c) Ruthenium-Komplex (IAc) der allgemeinen Formel (IAc)

   [Ru(L)XₚY₂₋ₚ]₂ Z₂ₚ (IAc)

   mit p = 0 oder 1;
(d) Ruthenium-Komplex (IAd) der allgemeinen Formel (IAd)

   [Ru(L)Xₚ₊₁Y₁₋ₚ]₂ Z₂ₚ (IAd)

   mit p = 0 oder 1;
(e) Ruthenium-Komplex (IAe) der allgemeinen Formel (IAe)

   [Ru(L)XₚY₃₋ₚ] Zₚ (IAe)

   mit p = 0 oder 1;
(f) Ruthenium-Komplex (lAf) der allgemeinen Formel (lAf)

   [Ru(L)XₚY₂₋ₚ] Z₁₊ₚ (lAf)

   mit p = 0 oder 1; und
(g) Ruthenium-Komplex (lAg) der allgemeinen Formel (lAg)

   [Ru(L)Xₚ₋₁Y₃₋ₚ]₂ Z₂ₚ (lAg)

   mit p = 1;
(h) Ruthenium-Komplex (IAh) der allgemeinen Formel (IAh)

   [Ru(L)XₚY₂₋ₚ]₂ Z₂ₚ (IAh)

   mit p = 1.

Als bevorzugte Beispiele zu Ruthenium-Komplex (IAa) seien genannt [Ru(L)(PPh₃)Cl₂], [Ru(L)(PPh₃)Cl(OAc)], [Ru(L)(PPh₃)(H)(Cl)], [Ru(L)(PPh₃)(OAc)₂], [Ru(L)(PPh₃)acac(H)], [Ru(L)(PPh₃)(H)(OMe)], [Ru(L)(PPh₃)(H)₂], [Ru(L)(CO)(H)₂] [Ru(L)(PPh₃)(H)(OAlkyl)], [Ru(L)(PPh₃)(H)(OAc)], [Ru(L)(P(o-Tolyl)₃)Cl₂], [Ru(L)(P(o-Tolyl)₃)Cl(OAc)], [Ru(L)(P(o-Tolyl)₃)(H)(Cl)], [Ru(L)(P(o-Tolyl)₃)(OAc)₂], [Ru(L)(P(o-Tolyl)₃)acac(H)], [Ru(L)(P(o-Tolyl)₃)(H)(OMe)], [Ru(L)(P(o-Tolyl)₃)(H)]OMe, [Ru(L)(P(o-Tolyl)₃)(H)₂], [Ru(L)(P(o-Tolyl)₃)(H)(OAlkyl)], [Ru(L)(P(o-Tolyl)₃)(H)(OAc)], [Ru(L)(P(p-Tolyl)₃)(Cl)₂], [Ru(L)(P(p-Tolyl)₃)Cl(OAc)], [Ru(L)(P(p-Tolyl)₃)(H)(Cl)], [Ru(L)(P(p-Tolyl)₃)(OAc)₂], [Ru(L)(P(p-Tolyl)₃)acac(H)], [Ru(L)(P(p-Tolyl)₃)(H)(OMe)], [Ru(L)(P(p-Tolyl)₃)H₂], [Ru(L)(P(p-Tolyl)₃)(H)(OAlkyl)], [Ru(L)(P(p-Tolyl)₃)(H)(OAc)], [Ru(L)(PCy₃)Cl₂], [Ru(L)(PCy₃)Cl(OAc)], [Ru(L)(PCy₃)(H)(Cl)], [Ru(L)(PCy₃)(OAc)₂], [Ru(L)(PCy₃)acac(H)], [Ru(L)(PCy₃)(H)(OMe)], [Ru(L)(PCy₃)H₂], [Ru(L)(PCy₃)(H)(OAl-kyl)], [Ru(L)(PCy₃)(H)(OAc)], [Ru(L)(PtBu₃)Cl₂], [Ru(L)(PtBu₃)Cl(OAc)], [Ru(L)(PtBu₃)(H)(Cl)], [Ru(L)(PtBu₃)(OAc)₂], [Ru(L)(PtBu₃)acac(H)], [Ru(L)(PtBu₃)(H)(OMe)], [Ru(L)(PtBu₃)(H)₂], [Ru(L)(PtBu₃)(H)(OAlkyl)], [Ru(L)(PtBu₃)(H)(OAc)], [Ru(L)(P(OR)₃)Cl₂], [Ru(L)(CO)(H)(CI)], [Ru(L)(CO)(H)(OAlk)], [Ru(L)(CO)(H)(OMe)], [Ru(L)(P(OR)₃)(H)₂], [Ru(L)(P(OR)₃)(H)(Cl)], [Ru(L)(P(OR)₃)(OAc)₂], wobei R bevorzugt für Methyl, Ethyl, iso-Propyl, iso-Butyl, tert.-Butyl, Phenyl, o-Tolyl, p-Tolyl, 2,4-Dimethylphenyl oder 2,4-Di-tert.-butylphenyl steht, [Ru(L)(P(OR)₃)acac], [Ru(L)(NHC)Cl₂], [Ru(L)(NHC)(OAc)₂], [Ru(L)(NHC)acac], [Ru(L)(PPh₃)(CO)(H)]Cl, [Ru(L)(PPh₃)(CO)(H)]OAc, [Ru(L)(P(o-Tolyl)₃)(CO)(H)]Cl, [Ru(L)(P(o-Tolyl)₃)(CO)(H)]OAc, [Ru(L)(P(p-Tolyl)₃)(CO)(H)]Cl, [Ru(L)(P(p-Tolyl)₃)(CO)(H)]OAc, [Ru(L)(PCy₃)(CO)(H)]Cl, [Ru(L)(PCy₃)(CO)(H)]OAc, [Ru(L)(PtBu₃)(CO)(H)]Cl, [Ru(L)(PtBu₃)(CO)(H)]OAc und [Ru(L)(CO)Cl₂], wobei L jeweils für die neutralen Liganden (Ila), (IIb) oder (IIc) steht.

Als bevorzugte Beispiele zu Ruthenium-Komplex (lAb) seien genannt [Ru(L)H₂], [Ru(L)Cl₂], [Ru(L)OAc₂], [Ru(L)H(OMe)], [Ru(L)H(OAlk)], [Ru(L)(H)acac], [Ru(L)(H)(CI)], [Ru(L)(H)OAc], [Ru(L)(PPh₃)OAc]Cl, [Ru(L)(PPh₃)(OMe)]Cl, [Ru(L)(PPh₃)(OMe)]OAc, [Ru(L)(PPh₃)(H)]OAc, [Ru(L)(PPh₃)(H)]OMe, [Ru(L)(CO)(H)]OMe, [Ru(L)(CO)(H)]OAc und [Ru(L)(PPh₃)Cl]OAc, wobei L jeweils für die neutralen Liganden (IIa), (IIb) oder (IIc) steht.

Als bevorzugte Beispiele zu Ruthenium-Komplex (IAc) seien genannt [Ru(L)(PPh₃)Cl]₂Cl₂, [Ru(L)(Cl)₂]₂, [Ru(L)(OMe)₂]₂ ), wobei L jeweils für die neutralen Liganden (IIa), (IIb) oder (llc) steht, oder auch [Ru(L)(OMe)₂]₂, [Ru(L)(Cl)₂]₂, wobei L jeweils für die anionischen Liganden (Ild) steht.

Als bevorzugte Beispiele zu Ruthenium-Komplex (IAd) seien genannt [Ru(L)(H)(PPh₃)]₂, [Ru(L)(H)(CO)]₂, [Ru(L)(Cl)(CO)]₂, [Ru(L)(Cl)(PPh₃)]₂, [Ru(L)(OMe)(CO)]₂, wobei L jeweils für die anionischen Liganden (IId) steht.

Als bevorzugte Beispiele zu Ruthenium-Komplex (IAe) seien genannt [Ru(L)(PPh₃)(Cl)₂]BF₄, [Ru(L)(PPh₃)(Cl)₂]PF₆, [Ru(L)(PPh₃)(Cl)₂]OAc, [Ru(L)(PPh₃)(Cl)₂]acac, [Ru(L)(PPh₃)(H)(Cl)]BF₄, [Ru(L)(PPh₃)(H)(Cl)]PF₆, [Ru(L)(PPh₃)(H)(Cl)]OAc, [Ru(L)(PPh₃)(H)(Cl)]OMe, [Ru(L)(CO)(H)(Cl)]BF₄, [Ru(L)(CO)(H)(Cl)]PF₆, [Ru(L)(CO)(H)(CI)]OAc, [Ru(L)(CO)(H)(CI)]OMe, [Ru(L)(CO)(Cl)₂]BF₄, [Ru(L)(CO)(Cl)2]PF₆, [Ru(L)(CO)(Cl)₂]OAc, [Ru(L)(CO)(Cl)₂]OMe, [Ru(L)(OAc)₂]BF₄, [Ru(L)(OAc)₂]PF₆ und [Ru(L)(OAc)₂] OAc, wobei L jeweils für die neutralen Liganden (IIa), (IIb) oder (llc) steht.

Als bevorzugte Beispiele zu Ruthenium-Komplex (lAf) seien genannt [Ru(L)(PPh₃)(CF₃SO₃)](CF₃SO₃)₂, [Ru(L)(CO)(Cl)](OAc)₂, [Ru(L)(OAc)₂](OAc) und [Ru(L)(Cl)₂](Cl), wobei L jeweils für die neutralen Liganden (IIa), (IIb) oder (llc) steht.

Als bevorzugte Beispiele zu Ruthenium-Komplex (lAg) seien genannt [Ru(L)(Cl)₂]₂(BF₄)₂ und [Ru(L)(Cl)₂]₂(PF₆)₂ wobei L jeweils für die neutralen Liganden (IIa), (IIb) oder (llc) steht.

Als bevorzugte Beispiele zu Ruthenium-Komplex (IAh) seien genannt [Ru(L)(PPh₃)(Cl)]₂(BF₄)₂, [Ru(L)(PPh₃)(Cl)]₂(PF₆)₂, [Ru(L)(PPh₃)(Cl)]₂(OAc)₂, [Ru(L)(PPh₃)(Cl)]₂(acac)₂, [Ru(L)(CO)(Cl)]₂(BF₄)₂, [Ru(L)(CO)(Cl)]₂(PF₆)₂, [Ru(L)(CO)(Cl)]₂(OAc)₂, [Ru(L)(CO)(Cl)]₂(acac)₂, wobei L jeweils für die anionischen Liganden (Ild) steht.

Besonders bevorzugte Ruthenium-Komplexe (IA) sind die Ruthenium-Komplexe (IAa) und (IAc).

Ganz besonders bevorzugt führt man das erfindungsgemäße Verfahren in Gegenwart von Ruthenium-Komplexen (I) durch, bei denen
- der Ligand (II) für den Liganden (Ila), (IIb) oder (IIc) steht und darin
- die Reste R¹, R² jeweils für Phenyl, p-Tolyl, 3,5-Dimethyl-4-methoxyphenyl, iso-Butyl oder Cyclohexyl stehen,
- die Reste R⁵ und R¹⁰ unabhängig voneinander für Wasserstoff, Methyl oder tert.-Butyl stehen,
- der Rest R¹¹ unabhängig voneinander für Wasserstoff, Methyl oder Methoxy steht,
- die Reste R⁷, R⁸ und R⁹ unabhängig voneinander für Wasserstoff oder Methyl stehen, und
- der Ruthenium-Komplexe (I) die Zusammensetzung [Ru(L)(PPh₃)Cl₂], [Ru(L)(PPh₃)(H)(Cl)], [Ru(L)(PPh₃)(OAc)₂], [Ru(L)(PPh₃)H(acac)], [Ru(L)(PPh₃)(H)(OMe)], [Ru(L)(PPh₃)(H)]OMe, [Ru(L)(P(o-Tolyl)₃)Cl₂], [Ru(L)(P(o-Tolyl)₃)Cl(OAc)], [Ru(L)(P(o-Tolyl)₃)(H)(Cl)], [Ru(L)(P(o-Tolyl)₃)(OAc)₂], [Ru(L)(P(o-Tolyl)₃)acac], [Ru(L)(P(o-Tolyl)₃)(H)(OMe)], [Ru(L)(P(p-Tolyl)₃)Cl₂], [Ru(L)(P(p-Tolyl)₃)Cl(OAc)], [Ru(L)(P(p-Tolyl)₃)(H)(Cl)], [Ru(L)(P(p-Tolyl)₃)(OAc)₂], [Ru(L)(P(p-Tolyl)₃)acac], [Ru(L)(P(p-Tolyl)₃)(H)(OMe)], [Ru(L)(PCy₃)Cl₂], [Ru(L)(PCy₃)Cl(OAc)], [Ru(L)(PCy₃)(H)(Cl)], [Ru(L)(PCy₃)(OAc)₂], [Ru(L)(PCy₃)acac], [Ru(L)(PCy₃)(H)(OMe)], [Ru(L)(PtBu₃)Cl₂], [Ru(L)(PtBu₃)Cl(OAc)], [Ru(L)(PtBu₃)(H)(Cl)], [Ru(L)(PtBu₃)(OAc)₂], [Ru(L)(PtBu₃)acac], [Ru(L)(PtBu₃)(H)(OMe)], [Ru(L)(P(OR)₃)(OAc)₂], [Ru(L)H(OMe)], [Ru(L)H(OAlk)], [Ru(L)(P(OR)₃)acac], [Ru(L)(NHC)Cl₂], [Ru(L)(NHC)(OAc)₂], [Ru(L)(NHC)acac], [Ru(L)(PPh₃)OAc]Cl, [Ru(L)(PPh₃)(OMe)]Cl, [Ru(L)(PPh₃)(OMe)]OAc, [Ru(L)(PPh₃)Cl]OAc, [Ru(L)(PPh₃)(Cl)]₂OAc₂, [Ru(L)(PPh₃)(Cl)]₂Cl₂, [Ru(L)(Cl)₂]₂, [Ru(L)(OAc)₂]₂, [Ru(L)(OMe)₂]₂, [Ru(L)(H)(Cl)]₂, oder [Ru(L)(H)(OAc)]₂ aufweist.

Die beim erfindungsgemäßen Verfahren einzusetzenden Ruthenium-Komplexe (I) können auf verschiedene Art und Weise erhalten werden. Eine bevorzugte Möglichkeit besteht darin, als Ruthenium-haltigen Ausgangsstoff eine Verbindung einzusetzen, in der das Ruthenium bereits in Form eines Komplexes vorliegt, im Folgenden als Ru-Precursorkomplex (IV) bezeichnet, und diesen mit dem Liganden L umsetzt. Entsprechend ist ein Verfahren bevorzugt, bei dem man den Ruthenium-Komplex (I) durch Umsetzung von Ligand (II) mit einem Ru-Precursorkomplex (IV) erhält.

Als Ru-Precursorkomplex (IV) können grundsätzlich die verschiedensten Ru-Komplexe eingesetzt werden. Üblicherweise handelt es sich beim Ru-Precursorkomplex (IV) um einen fünffach oder sechsfach koordinierten Ruthenium-Komplex, welcher auch zu einem Dimer oder Trimer verbrückt sein kann. Die Oxidationsstufe des Rutheniums beträgt bevorzugt 0, +2 oder +3. Entsprechend enthält der Ru-Precursorkomplex (IV) neutrale und/oder anionische Liganden sowie falls zur Erreichung der Gesamtladung "0" erforderlich ein oder mehrere nicht-koordinierende Anionen. Dabei ist es in vielen Fällen nicht erforderlich, dass der Ru-Precursorkomplex (IV) bereits die Liganden X und Y sowie das nicht-koordinierende Anion Y des gewünschten Ruthenium-Komplexe (I) enthält. Die Liganden X und Y sowie das nicht-koordinierende Anion Y können in vielen Fällen dem Syntheseansatz auch separat zugeführt werden. Um den Syntheseaufwand niedrig zu halten, setzt man vorteilhafterweise als Ru-Precursorkomplexe (IV) leicht zugängliche beziehungsweise leicht verfügbare Komplexe ein. Dem Fachmann sind derartige Komplexe wohlbekannt. Ebenso ist der Fachmann auch mit dem Austausch von Liganden an Ruthenium-haltigen Komplexen vertraut.

Als neutrale Liganden im Ru-Precursorkomplex (IV) kommen grundsätzlich alle neutralen Liganden in Frage, welche bereits unter dem Liganden X beschrieben sind, wobei natürlich auch im Ru-Precursorkomplex (IV) zwei Liganden X zu einem zweizähnigen Liganden miteinander verbunden sein können. Zudem kommen noch weitere, nicht unter X genannte neutrale Liganden in Frage. Als Beispiel hierzu sei etwa Benzol und p-Cymol genannt. Als bevorzugte neutrale Liganden im Ru-Precursorkomplex (IV) sind Triphenylphosphin, CO und Cycloocta-1,5-dien genannt.

Als anionische Liganden im Ru-Precursorkomplex (IV) kommen grundsätzlich alle anionischen Liganden in Frage, welche bereits unter dem Liganden Y beschrieben sind, wobei natürlich auch im Ru-Precursorkomplex (IV) der anionische Ligand Y zweizähnig sein kann. Zusätzlich kommen noch weitere, nicht unter Y genannte anionische Liganden in Frage. Als Beispiel hierzu sei etwa Methylallyl genannt. Als bevorzugte anionische Liganden im Ru-Precursorkomplex (IV) sind Cl-, Acetylacetonato und Methylallyl genannt.

Die Umsetzung des Ru-Precursorkomplexes (IV) mit dem Liganden L führt man üblicherweise bei einem Ru/L-Molverhältnis von 0,8 bis 20 bevorzugt von 0,9 bis 10 und besonders bevorzugt von 0,9 bis 1,1 durch. Im Sinne eines möglichst hohen Umsetzungsgrades ist es dabei vorteilhaft, einen Ru-Precursorkomplex (IV) mit nur ein- und zweizähnigen Liganden einzusetzen, um den Komplexierungseffekt des dreizähnigen Liganden L zu nutzen. Üblicherweise führt man die Umsetzung wasserfrei, jedoch in Gegenwart eines Lösungsmittels und unter einer Schutzgasatmosphäre durch. Als geeignete Lösungsmittel seien beispielhaft aliphatische Alkohole wie beispielsweise Methanol, Ethanol oder Isopropanol sowie aromatische Kohlenwasserstoffe wie etwa Toluol oder Xylole genannt. Im Allgemeinen weißt Ruthenium im Ru-Precursorkomplex (IV) dieselbe Oxidationsstufe auf wie im anschließenden Ruthenium-Komplexe (I), und bevorzugt somit die Oxidationsstufe +2 oder +3.

Der Ruthenium-Komplexe (I) kann aus dem erhaltenen Reaktionsgemisch beispielsweise durch Ausfällen oder Kristallisation isoliert werden.

Zur Durchführung der erfindungsgemäßen Hydrierung ist es jedoch im Allgemeinen nicht erforderlich, den Ruthenium-Komplexe (I) nach dessen Herstellung erst zu isolieren. Vielmehr ist es im Sinne einer vereinfachten Verfahrensführung vorteilhaft, den Ruthenium-Komplexe (I) wie oben beschrieben aus einem Ru-Precursorkomplex (IV) und dem Liganden L in Gegenwart eines Lösungsmittels herzustellen und die erfindungsgemäße Hydrierung direkt in dem erhaltenen Reaktionsgemisch durchzuführen.

Die beim erfindungsgemäßen Verfahren einzusetzenden Ester können vielfältiger Natur sein. So können grundsätzlich lineare oder verzweigte, nichtcyclische oder cyclische, gesättigte oder ungesättigte, aliphatische, aromatische oder araliphatische, unsubstituierte oder durch Heteroatome oder funktionelle Gruppen unterbrochene Ester unterschiedlicher Molmassen von niedermolekular bis hochmolekular eingesetzt werden.

Bevorzugt setzt man als Ester einen Ester der allgemeinen Formel (III) 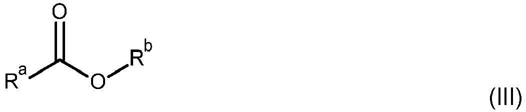 ein, bei dem die Reste R^{a} und R^{b} jeweils unabhängig voneinander für einen Kohlenstoff enthaltenden organischen, linearen oder verzweigten, nichtcyclischen oder cyclischen, gesättigten oder ungesättigten, aliphatischen, aromatischen oder araliphatischen, unsubstituierten oder durch Heteroatome oder funktionelle Gruppen unterbrochenen oder substituierten Rest mit einer Molmasse von 15 bis 10000 g/mol stehen, wobei beide Reste R^{a} und R^{b} auch miteinander verbunden sein können.

Im Falle von verzweigten Resten R^{a} und R^{b} können diese einfach oder mehrfach verzweigt sein. Ebenso können im Falle von cyclischen Resten, diese einfach oder mehrfach cyclisch sein. Desgleichen können im Falle von ungesättigten Resten diese einfach oder mehrfach ungesättigt sein, wobei hierbei sowohl Doppelbindungen als auch Dreifachbindungen möglich sind. Als Heteroatome sind Atome zu verstehen, welche weder Kohlenstoff noch Wasserstoff sind. Als bevorzugte Beispiele von Heteroatomen seien genannt Sauerstoff, Stickstoff, Schwefel, Phosphor, Fluor, Chlor, Brom und lod, sowie als besonders bevorzugte Beispiele Sauerstoff, Stickstoff, Fluor, Chlor und Brom. Funktionelle Gruppen sind eine weitere Umschreibung von Gruppen, welche mindestens ein Heteroatom enthalten. So kann beispielsweise eine mittels -O- unterbrochene Kohlenwasserstoffkette sowohl als eine Kohlenwasserstoffkette betrachtet werden, die durch ein Sauerstoff-Heteroatom unterbrochen ist, als auch als eine Kohlenwasserstoffkette, die durch eine Ether-Gruppe unterbrochen ist. Als weitere, nicht einschränkende Beispiele seien etwa Amino-Gruppen (-NH₂, -NH-, -N<), AldehydGruppen (-CHO), Carboxy-Gruppen (-COOH), Amid-Gruppen (-CONH₂, -CONH-, -CON<), Nitril-Gruppen (-CN), Isonitril-Gruppen (-NC), Nitro-Gruppen (-NO₂), Sulfonsäure-Gruppen (-SO₃), Keto-Gruppen (>CO), Imino-Gruppen (>CNH, >CN-), Ester-Gruppen (-CO-O-), Anhydrid-Gruppen (-CO-O-CO-) und Imido-Gruppen (-CO-NH-CO-, -CO-NR-CO-) genannt. Es können natürlich auch mehrere sogenannter funktioneller Gruppen enthalten sein. Als Beispiel hierfür seien etwa Fette genannt.

Sind die Reste R^{a} und R^{b} miteinander verbunden, liegen cyclische Ester vor, die auch als Lactone bezeichnet werden.

Die Molmassen der Reste R^{a} und R^{b} betragen im Allgemeinen 15 bis 10000 g/mol, bevorzugt 15 bis 5000 g/mol und besonders bevorzugt 15 bis 2000 g/mol.

Bevorzugt setzt man beim erfindungsgemäßen Verfahren Ester mit einer Molmasse von 74 bis 20000 g/mol, besonders bevorzugt von 74 bis 10000 g/mol, ganz besonders bevorzugt von 74 bis 5000 g/mol, vor allem 74 bis 2000 g/mol und insbesondere von 74 bis 1000 g/mol ein.

Der beim erfindungsgemäßen Verfahren einzusetzende molekulare Wasserstoff (H₂) kann sowohl unverdünnt als auch mit Inertgas, beispielsweise Stickstoff, verdünnt zugeführt werden. Vorteilhaft ist die Zufuhr eines Wasserstoff-enthaltenden Gases mit einem möglichst hohen Gehalt an Wasserstoff. Bevorzugt ist ein Gehalt an Wasserstoff von ≥ 80 Vol.-%, besonders bevorzugt von ≥ 90 Vol.-%, ganz besonders bevorzugt von ≥ 95 Vol.-% und insbesondere von ≥ 99 Vol.-%.

Beim erfindungsgemäßen Verfahren führt man in einer ganz allgemeinen Ausführungsform einem geeigneten Reaktionsapparat den Ruthenium-Komplex (I), den zu hydrierenden Ester sowie Wasserstoff zu und setzt das Gemisch unter den gewünschten Reaktionsbedingungen um.

Als Reaktionsapparate können beim erfindungsgemäßen Verfahren grundsätzlich Reaktionsapparate eingesetzt werden, die für gas/flüssig-Reaktionen unter der gegebenen Temperatur und dem gegebenen Druck grundsätzlich geeignet sind. Geeignete Standardreaktoren für gas/flüssig- und für flüssig/flüssig-Reaktionssyteme sind beispielsweise in K.D. Henkel, "Reactor Types and Their Industrial Applications", in UII-mann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" beschrieben. Als Beispiele seien genannt Rührkesselreaktoren, Rohrreaktoren oder Blasensäulenreaktoren. Druckresistente Rührkessel werden in der Regel auch als Autoklaven bezeichnet.

Der Ruthenium-Komplex (I) kann dabei direkt in Form des vorab synthetisierten Ruthenium-Komplexes (I) dem Reaktionsapparat zugeführt werden. Dies bedarf jedoch einer vorausgegangenen Synthese mit anschließender Aufarbeitung beziehungsweise Isolierung und Handhabung unter Schutzgasatmosphäre.

Deutlich einfacher und bevorzugt ist es, dass man den Ruthenium-Komplex (I) in-situ aus einem Ru-Precursorkomplex (IV) und Ligand L (II) bildet. In-situ steht für "vor Ort" und bedeutet, dass man den Ruthenium-Komplex (I) durch Zufuhr von Ru-Precursorkomplex (IV) und Ligand L (II) im Reaktionsapparat bildet. Vorteilhafterweise setzt man hierzu ein Molverhältnis von Ligand L (II) zu Ruthenium von 0,5 bis 5, bevorzugt ≥ 0,8 und besonders bevorzugt ≥ 1, sowie bevorzugt ≤ 3, besonders bevorzugt ≤ 2 und ganz besonders bevorzugt ≤ 1,5 ein. Diese in-situ Variante erspart das vorherige Isolieren des Liganden (II).

Eine weitere, noch deutlich einfachere Möglichkeit zur in-situ Darstellung des Ruthenium-Komplexes (I) besteht darin, dass man den Ruthenium-Komplex (I) ohne Isolierung oder Aufreinigung aus einem Ru-Precursorkomplex (IV) und den Synthesebausteinen des Liganden L (II) bildet. Aus den Synthesebausteinen des Liganden L (II) bildet sich zunächst der Ligand (II), welcher am Ruthenium koordiniert und zum Ruthenium-Komplex (I) führt. Besonders bevorzugt ist es daher, dass man den Ruthenium-Komplex (I) in-situ durch Umsetzung
(a) eines Aldehyds oder Ketons der allgemeinen Formel (Va) 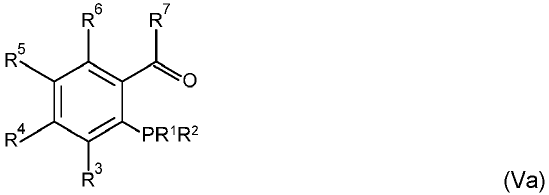 mit einem Amin der allgemeinen Formel (Vb) 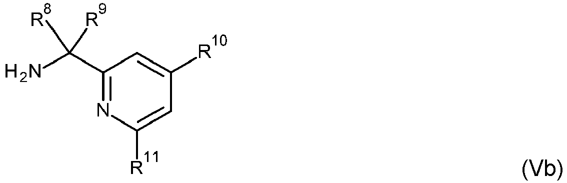 und/oder
(b) eines Amins der allgemeinen Formel (VIa) 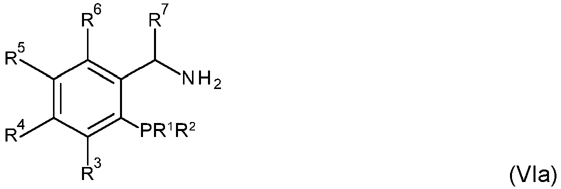 mit einem Aldehyd oder einem Keton der allgemeinen Formel (Vlb) 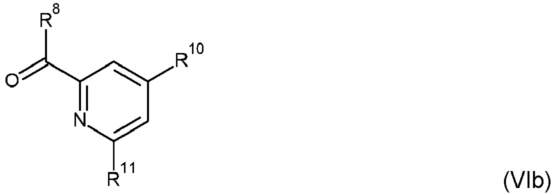 zum Liganden L (II), wobei die Reste R¹ bis R¹¹ jeweils die zuvor definierte Bedeutung haben, und nachfolgender Umsetzung des gebildeten Liganden L (II) ohne dessen Isolierung oder Aufreinigung mit einem Ru-Precursorkomplex (IV) bildet.

Das erfindungsgemäße Verfahren kann in Gegenwart aber auch in Abwesenheit eines Lösungsmittels erfolgen. Wird ein Lösungsmittel eingesetzt, so dient dieses beispielsweise zur Lösung des Ruthenium-Komplexes (I) beziehungsweise eines Ru-Precursorkomplexes (IV) sowie des Liganden L, aber auch gegebenenfalls zur Lösung des zu hydrierenden Esters. Vor allem bei niedermolekularen Estern kann auch dieser als Lösungsmittel fungieren.

Werden Lösungsmittel eingesetzt, so kommen bevorzugt Lösungsmittel mit mehr oder minder stark ausgeprägten polaren Eigenschaften in Frage, die unter den Reaktionsbedingungen nicht selbst hydriert werden. Als bevorzugte Beispiele seien genannt aliphatische Alkohole wie beispielsweise Methanol, Ethanol oder Isopropanol sowie aromatische Kohlenwasserstoffe wie etwa Toluol oder Xylole. Die Menge an eingesetztem Lösungsmittel kann breit variieren. Üblich sind jedoch Mengen im Bereich von 0,1 bis 20 g Lösungsmittel pro g zu hydrierendem Ester, bevorzugt 0,5 bis 10 g Lösungsmittel pro g zu hydrierendem Ester und besonders bevorzugt 1 bis 5 g Lösungsmittel pro g zu hydrierendem Ester.

Der zu hydrierende Ester kann direkt in Form des reinen, unverdünnten Esters, aber auch verdünnt beziehungsweise in einem Lösungsmittel gelöst zugeführt werden. Kriterien, in welcher Form der zu hydrierende Ester zugegeben wird, sind in der Regel oftmals rein praktischer Natur, wie beispielsweise die Natur des vorliegenden Esters sowie dessen Handhabung. So ist es beispielsweise anzustreben, dass das der Ester im Reaktionsgemisch unter den Reaktionsbedingungen in flüssiger Form vorliegt.

Das Molverhältnis zwischen dem zu hydrierenden Ester und dem Ruthenium-Komplex (I) kann beim erfindungsgemäßen Verfahren in einem breiten Bereich variieren. Im Allgemeinen beträgt das genannte Molverhältnis in dem zu hydrierenden Reaktionsgemisch 1 bis 100000, bevorzugt 10 bis 25000, besonders bevorzugt 100 bis 5000 und ganz besonders bevorzugt 500 bis 20000.

Das erfindungsgemäße Verfahren führt man bei einer Temperatur von 50 bis 200°C, bevorzugt bei ≤ 170°C und besonders bevorzugt bei ≤ 150°C durch. Der Druck beträgt dabei 0,1 bis 20 MPa abs, bevorzugt ≥ 1 MPa abs und besonders bevorzugt ≥ 5 MPa abs, sowie bevorzugt ≤ 15 MPa abs und besonders bevorzugt ≤ 10 MPa abs.

Die Reaktionszeit beziehungsweise mittlere Verweilzeit, in der das Reaktionsgemisch unter den Reaktionsbedingungen vorliegt, kann ebenfalls breit variieren, liegt jedoch üblicherweise im Bereich von 0,1 bis 100 Stunden, bevorzugt bei ≥ 1 Stunden und besonders bevorzugt bei ≥ 2 Stunden, sowie bevorzugt bei ≤ 80 Stunden und besonders bevorzugt bei ≤ 60 Stunden.

Des Weiteren hat sich gezeigt, dass in der Regel die erfindungsgemäße Hydrierung durch Gegenwart einer Base positiv beeinflusst wird und dadurch letztendlich deutlich höhere Umsätze ermöglicht werden. Daher ist es in den meisten Fällen vorteilhaft, die Hydrierung in Gegenwart einer Base durchzuführen. In Ausnahmefällen, bei denen beispielsweise das Edukt basenlabil ist oder mit Base unter Reaktionsbedingungen Nebenreaktionen einsetzen, kann eine Reaktionsführung in der Gesamtschau auch ohne Base vorteilhafter sein. Grundsätzlich können die Basen im Reaktionsgemisch auch als Feststoff vorliegen, bevorzugt sind jedoch Basen, die im Reaktionsgemisch gelöst vorliegen. Als Beispiele möglicher Basen seien genannt Alkoholate, Hydroxide, Alkalimetall- und Erdalkalimetall-Carbonate, Amide, basische Aluminium und Silicium Verbindungen sowie Hydride. Besonders bevorzugt setzt man als Base Alkoholate oder Amide, bevorzugt Natriummethanolat, Kaliummethanolat, Natriumhydroxid, Natriumborhydrid oder Natriumhydrid, und insbesondere Natriummethanolat und Kaliummethanolat ein.

Wird das erfindungsgemäße Verfahren in Gegenwart einer Base durchgeführt, so wird diese in Bezug auf den Ruthenium-Komplex (I) im Allgemeinen im Überschuss eingesetzt. Bevorzugt setzt man ein Molverhältnis der Base zum Ruthenium-Komplex (I) von 2 bis 1000, bevorzugt von ≥ 10, besonders bevorzugt von ≥ 20 und ganz besonders bevorzugt von ≥ 50, sowie bevorzugt von ≤ 500 und besonders bevorzugt von ≤ 250 ein.

Das erfindungsgemäße Verfahren kann kontinuierlich, in semibatch-Fahrweise, diskontinuierlich, rückvermischt in Produkt als Lösungsmittel oder im geraden Durchgang nicht rückvermischt durchgeführt werden. Die Zuleitung des Ruthenium-Komplexes, des zu hydrierenden Esters, des Wasserstoffs, gegebenenfalls des Lösungsmittels und gegebenenfalls der Base kann dabei simultan oder getrennt voneinander erfolgen.

Bei der diskontinuierlichen Fahrweise legt man üblicherweise den Ruthenium-Komplex (I) beziehungsweise einen Ru-Precursorkomplex (IV) sowie den Liganden L (II), den zu hydrierenden Ester und gegebenenfalls Lösungsmittel und eine Base im Reaktionsapparat vor und stellt unter den gewünschten Reaktionsbedingungen unter Durchmischung durch Zugabe von Wasserstoff den gewünschten Reaktionsdruck ein. Anschließend belässt man das Reaktionsgemisch während der gewünschten Reaktionszeit unter den gewünschten Reaktionsbedingungen. Gegebenenfalls dosiert man Wasserstoff nach. Nach Ablauf der gewünschten Reaktionszeit kühlt man das Reaktionsgemisch ab beziehungsweise entspannt es. Durch eine nachfolgende Aufarbeitung können die korrespondierenden Alkohole als Reaktionsprodukt erhalten werden. Bevorzugt führt man die diskontinuierliche Umsetzung in einem Rührkessel durch.

Bei der kontinuierlichen Fahrweise führt man dem Reaktionsapparat den Ruthenium-Komplex (I) beziehungsweise einen Ru-Precursorkomplex (IV) sowie den Liganden L (II), den zu hydrierenden Ester und gegebenenfalls Lösungsmittel und eine Base kontinuierlich zu und entnimmt kontinuierlich eine entsprechende Menge zur Aufarbeitung und Isolierung des gebildeten korrespondierenden Alkohols.

Bevorzugt führt man die kontinuierliche Umsetzung in einem Rührkessel oder einer Rührkesselkaskade durch.

Das Hydrierprodukt lässt sich durch dem Fachmann an sich bekannte Verfahren, wie zum Beispiel durch Destillation und/oder Entspannungsverdampfung, aus dem Hydriergemisch abtrennen und der zurückbleibende Katalysator im Rahmen weiterer Umsetzungen nutzen. Im Rahmen der bevorzugten Ausführungsform verzichtet man vorteilhafterweise auf den Zusatz von Lösungsmitteln und führt die genannten Umsetzungen im umzusetzenden Substrat beziehungsweise dem Produkt und gegebenenfalls in hochsiedenden Nebenprodukten als Lösemedium durch. Insbesondere bevorzugt ist die kontinuierliche Reaktionsführung unter Wiederverwendung beziehungsweise Rückführung des homogenen Katalysators.

Bei der erfindungsmäßen Esterhydrierung werden aus der -CO-O- Estergruppe eine endständige -CH₂OH und eine endständige -OH Gruppe gebildet. Im Falle des Esters (III) bilden sich somit entsprechend der nachfolgenden Reaktionsgleichung die beiden korrespondierenden Alkohole R^{a}-CH₂OH und R^{b}-OH.

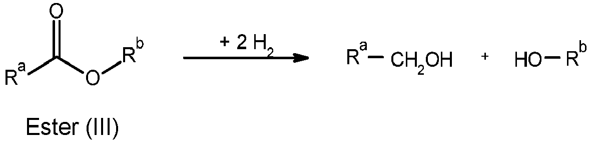

Beim Einsatz cyclischer Ester, sogenannter Lactone, sind die beiden Reste R^{a} und R^{b} miteinander verbunden und es bildet sich das entsprechende Diol.

Das erfindungsgemäße Verfahren ermöglicht die Herstellung von Alkoholen durch homogenkatalysierte Hydrierung von Estern in hoher Ausbeute und Selektivität. Die Hydrierung ist technisch in üblichem Laborgerät für Hydrierreaktionen durchführbar und ermöglicht den Einsatz der unterschiedlichsten Ester als Substrate.

Die besonderen Vorteile des erfindungsgemäßen Verfahrens basieren auf den speziellen, dreizähnigen PNN-Liganden. Durch seine Dreizähnigkeit koordiniert der Ligand fest am Ruthenium, hat aber im Vergleich zu vielen anderen dreizähnigen Liganden aus dem Stand der Technik eine eher niedrige Molmasse. So besitzt der erfindungsgemäße Ligand auch nur ein Phosphoratom, was sowohl bei den Herstellkosten als auch bei einer späteren Entsorgung von Vorteil ist. Der erfindungsgemäße Ligand liefert nach der Bildung entsprechender Ruthenium-Komplexe Katalysatoren mit hohen Hydrieraktivitäten. Zudem ist der erfindungsgemäße Ligand auch relativ oxidationsunempfindlich, so dass dieser auch in der Handhabung vorteilhaft ist und eine hohe Lagerstabilität aufweist.

Unter den besonderen Vorteilen des erfindungsgemäßen Liganden ist vor allem dessen leichte Zugänglichkeit und leichte Variationsmöglichkeit der Grundstruktur durch

Ersatz von Wasserstoffatomen durch diverse organische Reste zu nennen. Der Ligand kann in der Regel aus gut verfügbaren Einsatzstoffen durch eine einfache Eintopfsynthese hergestellt werden. Es ist in der Regel nicht erforderlich, den Liganden zu isolieren. Vielmehr kann sogar die Synthese des Liganden und die Darstellung des Ruthenium-Komplexes über eine Herstellung im Autoklaven direkt vor der Hydrierung (ohne Isolierung der Zwischenstufen) erfolgen. Als Ruthenium-haltigen Einsatzstoffe können leicht zugängliche und kommerziell in großen Mengen verfügbare Ruthenium-Precursorkomplexe eingesetzt werden.

### Beispiele

In den nachfolgenden Beispielen werden die in den Tabellen 1 bis 4 genannten Abkürzungen verwendet.

### Beispiel 1

In Beispiel 1 ist die Herstellung der verschiedenen Liganden in Form der Beispiele 1.1 bis 1.8 beschrieben.

### Beispiel 1.1 (Herstellung von Ligand 1 = L1)

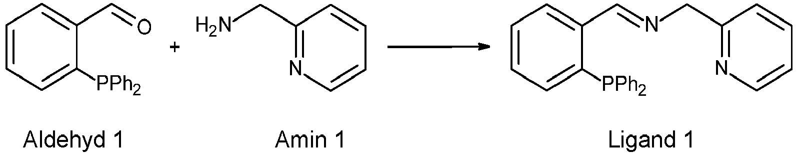

L1 wurde hergestellt wie beschrieben in Rigo et al. in Organometallics 2007, Vol. 26, Seiten 5636-5642.

³¹P NMR (203 MHz, CD₂Cl₂) δ -13.9.

### Beispiel 1.2 (Herstellung von Ligand 3 = L3)

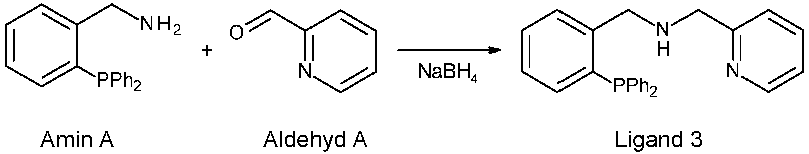

Bei Raumtemperatur wurde zu einer Lösung von Picolinaldehyd (Aldehyd A, 368 mg, 3,43 mmol) in Ethanol (10 mL) (2-(Diphenylphosphaneyl)phenyl)methanamin (Amin A, 1,00 g, 3,43 mmol) gegeben und das resultierende Gemisch 2 h bei Raumtemperatur gerührt. NaBH₄ (208 mg, 5,49 mmol) wurde zugegeben und weitere 2 h bei Raumtemperatur gerührt. Anschließend wurde wässrige gesättigte NaHCOs-Lösung (15 mL) und CH₂Cl₂ (25 mL) zugegeben. Nach Phasentrennung wurde die wässrige Phase mit CH₂Cl₂ (2 x 25 mL) extrahiert. Die vereinigte organische Phase wurde getrocknet (Na₂SO₄) und im Vakuum eingeengt. Das Rohprodukt wurde säulenchromatographisch an Kieselgel gereinigt (Hexan/EtOAc/NEt₃, 9:1 bis 1:1; es wurde eine Mischung von 10% NEt₃ in EtOAc verwendet) und N-(2-(Diphenylphosphaneyl)benzyl)-1-(pyridin-2-yl)methanamin **(L3)** als farbloses Öl (600 mg, 46% Ausbeute) erhalten.

¹H NMR (500 MHz, CD₂Cl₂) δ 8.48-8.46 (m, 1H), 7.57 (td, *J* = 7.7, 1.8 Hz, 1H), 7.54-7.51 (m, 1H), 7.36-7.30 (m, 7H), 7.28-7.24 (m, 4H), 7.19-7.10 (m, 4H), 6.91 (ddd, *J* = 7.7, 4.5, 1.4 Hz, 1H), 4.02 (d, *J =* 1.7 Hz, 2H), 3.79 (s, 2H). ³¹P NMR (203 MHz, CD₂Cl₂) δ -15.94. HRMS (ESI) C₂₅H₂₃N₂P ([M]⁺): Berechnet: 382.1599; Gefunden: 382.1611.

### Beispiel 1.3 (Herstellung von Ligand 4 = L4)

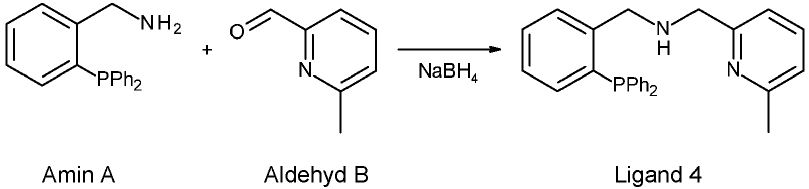

Bei Raumtemperatur wurde zu einer Lösung von 6-Methylpicolinaldehyd (Aldehyd B, 636 mg, 5,25 mmol) in Ethanol (20,0 mL) (2-(Diphenylphosphaneyl)phenyl)methana-min (Amin A, 1,53 g, 5,25 mmol) gegeben und das resultierende Gemisch 2 h bei Raumtemperatur gerührt. NaBH₄ (318 mg, 8,41 mmol) wurde zugegeben und weitere 2 h bei Raumtemperatur gerührt. Anschließend wurde wässrige gesättigte NaHCO₃-Lösung (50 mL) und CH₂Cl₂ (50 mL) zugegeben. Nach Phasentrennung wurde die wässrige Phase mit CH₂Cl₂ (2 x 25 mL) extrahiert. Die vereinigte organische Phase wurde getrocknet (Na₂SO₄) und in vacuo eingeengt. Das Rohprodukt wurde säulenchromatographisch an Kieselgel gereinigt (Hexan/EtOAc/NEt₃, 9:1 bis 6:4; es wurde eine Mischung von 10% NEt₃ in EtOAc verwendet) und N-(2-(Diphenylphospha-neyl)benzyl)-1-(6-methylpyridin-2-yl)methanamin **(L4)** als farbloses Öl (1,23 g, 3,10 mmol, 59% Ausbeute) erhalten.

¹H NMR (500 MHz, CD₂Cl₂) δ 7.54-7.52 (m, 1H), 7.46 (t, *J =* 7.7 Hz, 1H), 7.36-7.30 (m, 7H), 7.27-7.24 (m, 4H), 7.18-7.15 (m, 2H), 6.98 (d, *J* = 7.7 Hz, 1H), 6.94 (d, *J =* 7.7 Hz, 1H), 6.92-6.89 (m, 1H), 4.01 (s, 2H), 3.74 (s, 2H), 2.47 (s, 3H). ³¹P NMR (203 MHz, CD₂Cl₂) δ -16.31. HRMS (ESI) C₂₆H₂N₂P ([M]⁺): Berechnet: 396.1755; Gefunden: 396.1777.

### Beispiel 1.4 (Herstellung von Ligand 5 = L5)

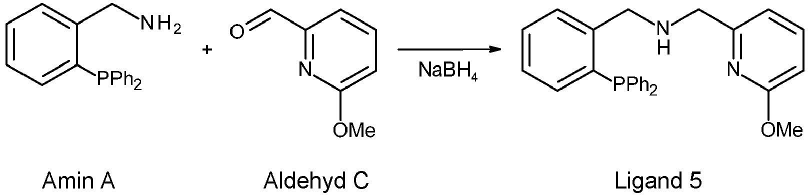

Bei Raumtemperatur wurde zu einer Lösung von 6-Methoxypicolinaldehyd (Aldehyd C, 706 mg, 5,14 mmol) in Ethanol (20 mL) (2-(Diphenylphosphaneyl)phenyl)methanamin (Amin A, 1,5 g, 5,14 mmol) gegeben und das resultierende Gemisch 2 h bei Raumtemperatur gerührt. NaBH₄ (311 mg, 8,22 mmol) wurde zugegeben und weitere 2 h bei Raumtemperatur gerührt. Anschließend wurde wässrige gesättigte NaHCO₃-Lösung (50 mL) und CH₂Cl₂ (50 mL) zugegeben. Nach Phasentrennung wurde die wässrige Phase mit CH₂Cl₂ (2 x 25 mL) extrahiert. Die vereinigte organische Phase wurde getrocknet (Na₂SO₄) und im Vakuum eingeengt. Das Rohprodukt wurde säulenchromatographisch an Kieselgel gereinigt (Hexan/EtOAc/NEt₃, 9:1 bis 6:4; es wurde eine Mischung von 10% NEt₃ in EtOAc verwendet) und N-(2-(Diphenylphosphaneyl)benzyl)-1-(6-methylpyridin-2-yl)methanamin **(L5)** als farbloses Öl (1,67 g, 4,06 mmol, 79% Ausbeute) erhalten.

¹H NMR (500 MHz, CD₂Cl₂) δ 7.56-7.51 (m, 1H), 7.46 (dd, J= 8.2, 7.2 Hz, 1H), 7.39-7.28 (m, 7H), 7.27-7.22 (m, 4H), 7.17 (td, J= 7.5, 1.4 Hz, 1H), 6.90 (ddd, J= 7.7, 4.4, 1.4 Hz, 1H), 6.71 (m, 1H), 6.56 (m, 1H), 4.01 (d, *J* = 1.8 Hz, 2H), 3.86 (s, 3H), 3.69 (s, 2H). ³¹P NMR (203 MHz, CD₂Cl₂) δ -16.25. HRMS (ESI) C₂₆H₂₅N₂P ([M]⁺): Berechnet: 396.1755; Gefunden: 396.1767.

### Beispiel 1.5 (Herstellung von Ligand 6 = L6)

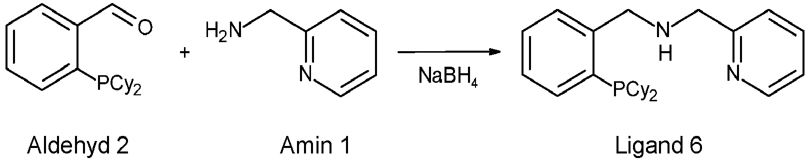

Bei Raumtemperatur wurde zu einer Lösung von 2-(Dicyclohexylphosphaneyl)benzaldehyd (Aldehyd 2, 2.00 g, 6.61 mmol) in Ethanol (50 mL) 2-Picolylamin (Amin 1, 715 mg, 6.61 mmol) gegeben und das resultierende Gemisch 2 h bei Raumtemperatur gerührt. NaBH4 (401 mg, 10.6 mmol) wurde zugegeben und weitere 2 h bei Raumtemperatur gerührt. Anschließend wurde wässrige gesättigte NaHCO₃-Lösung (100 mL) und CH₂Cl₂ (75 mL) zugegeben. Nach Phasentrennung wurde die wässrige Phase mit CH₂Cl₂ (2 x 50 mL) extrahiert. Die vereinigte organische Phase wurde getrocknet (Na₂SO₄) und im Vakuum eingeengt. Das Rohprodukt wurde säulenchromatographisch an Kieselgel gereinigt (Hexan/EtOAc/NEt₃, 9:1 bis 3:1; es wurde eine Mischung von 10% NEt₃ in EtOAc verwendet) und N-(2-(Dicyclohexylphosphaneyl)benzyl)-1-(pyridin-2-yl)methanamin **(L6)** als farbloses Öl (1.3 g, 54% Ausbeute) erhalten.

¹H NMR (500 MHz, C₆D₆) δ 8.50-8.49 (m, 1H), 7.51-7.79 (m, 1H), 7.44-7.41 (m, 1H), 7.24-7.22 (m, 1H), 7.18-7.17 (m, 1H), 7.14-7.10 (m, 2H), 6.65-6.62 (m, 1H), 4.31 (d, *J* = 2.1 Hz, 2H), 4.03 (s, 2H), 1.95-1.87 (m, 4H), 1.70-1.53 (m, 9H), 130.-1.00 (m, 11H). ³¹P NMR (203 MHz, CD₂Cl₂) δ -16.66. HRMS (ESI) C₂₆H₂₃N₂P ([M]⁺): Berechnet: 394.2538; Gefunden: 394.2527.

### Beispiel 1.6 (Herstellung von Ligand 7 = L7)

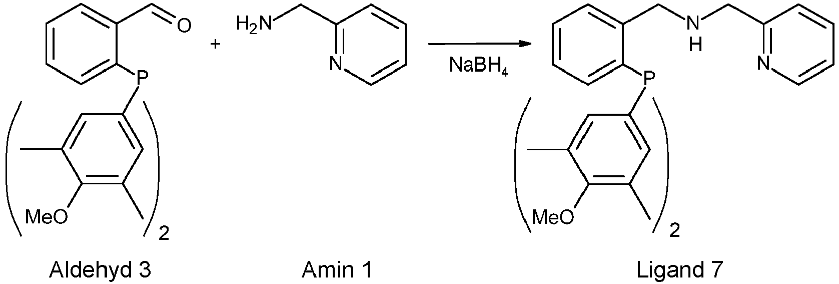

Bei Raumtemperatur wurde zu einer Lösung von 2-(Bis(4-methoxy-3,5-dimethylphenyl)phosphaneyl)benzaldehyd (Aldehyd 3, 2.00 g, 4.92 mmol) in Ethanol (50 mL) 2-Picolylamin (Amin 1, 532 mg, 4.92 mmol) gegeben und das resultierende Gemisch 2 h bei Raumtemperatur gerührt. NaBH₄ (300 mg, 7.88 mmol) wurde zugegeben und weitere 2 h bei Raumtemperatur gerührt. Anschließend wurde wässrige gesättigte NaHCOs-Lösung (50 mL) und CH₂Cl₂ (50 mL) zugegeben. Nach Phasentrennung wurde die wässrige Phase mit CH₂Cl₂ (2 x 25 mL) extrahiert. Die vereinigte organische Phase wurde getrocknet (Na₂SO₄) und im Vakuum eingeengt. Das Rohprodukt wurde säulenchromatographisch über Kieselgel gereinigt (Hexan/EtOAc/NEt₃, 9:1 bis 6:4; es wurde eine Mischung von 10% NEt₃ in EtOAc verwendet) und N-(2-(Bis(4-methoxy-3,5-dimethylphenyl)phosphaneyl)benzyl)-1-(pyridin-2-yl)methanamin **(L7)** als farbloses Öl (1.20 g, 50% Ausbeute) erhalten.

¹H NMR (500 MHz, C₆D₆) δ 8.45-8.44 (m, 1H), 7.63-7.60 (m 1H), 7.40 (ddd, J= 7.6, 4.4, 1.4 Hz, 1H), 7.27 (s, 2H), 7.25 (s, 2H), 7.18-7.17 (m, 1H), 7.09-7.05 (m, 2H), 7.00-6.98 (m, 1H), 6.63-6.60 (m, 1H), 4.26 (d, *J =* 1.9 Hz, 2H), 3.89 (s, 2H), 3.89 (s, 2H), 3.29 (s, 6H), 2.05 (s, 12H). ³¹P NMR (203 MHz, CD₂Cl₂) δ -17.13. HRMS (ESI) C₂₆H₂₅N₂P ([M]⁺): Berechnet: 498.2436; Gefunden: 498.2441.

### Generelle Arbeitsvorschriften 1-5 für die Hydrierungen

### Arbeitsvorschrift 1 (isolierter Ligand)

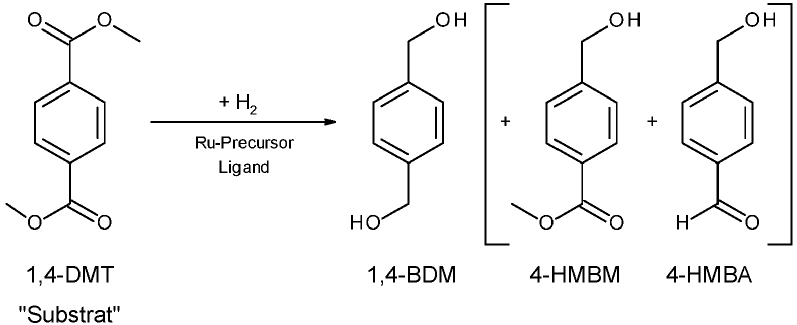

1,4-DMT = 1,4-Dimethylterephthalsäuremethylester
1,4-BDM = 1,4-Benzoldimethanol
4-HMBM = 4-Hydroxymethylbenzoesäuremethylester
4-HMBA = 4-Hydroxymethylbenzaldehyd

Unter Schutzgas wurden der ausgewählte Ligand (gemäß Angabe im jeweiligen Beispiel), der ausgewählte Ru-Precursor (gemäß Angabe im jeweiligen Beispiel), 1,4-Dimethylterephthalsäuremethylester (gemäß Angabe im jeweiligen Beispiel) und NaOMe (gemäß Angabe im jeweiligen Beispiel) in einem 100 mL-Autoklaven vorgelegt und mit 40 mL Toluol versetzt. Der Autoklav wurde verschlossen, ein Wasserstoffdruck von 6,0 MPa abs angelegt und bei 700 rpm auf die gewünschte Reaktionstemperatur erhitzt (gemäß Angabe im jeweiligen Beispiel). Nach Erreichen der gewünschten Reaktionstemperatur wurde ein Wasserstoffdruck von 8,0 MPa abs eingestellt. Nach Ablauf der gewünschten Reaktionszeit bei der gewünschten Reaktionstemperatur wurde der Autoklav auf Raumtemperatur abgekühlt, der erhaltene Austrag eingeengt, gegebenenfalls die Ausbeute bestimmt und der Austrag per GC analysiert (Auflösen einer Probe in Dioxan). Optima FFAP Säule (30 m x 0.25 mm / 0.5 µm; 15 min bei 140°C dann mit 20°C/min auf 250°C; Durchfluss: 2.0 mL/min; Wasserstoff als Trägergas). Umsatzbestimmung mittels GC-FI.%. t_{R}(1,4-BDM) = 24.9 min; t_{R}(4-HMBM) = 23.0 min, t_{R}(4-HMBA) = 22.5 min.

### Arbeitsvorschrift 2 (ohne Isolierung der Zwischenstufen des Liganden L)

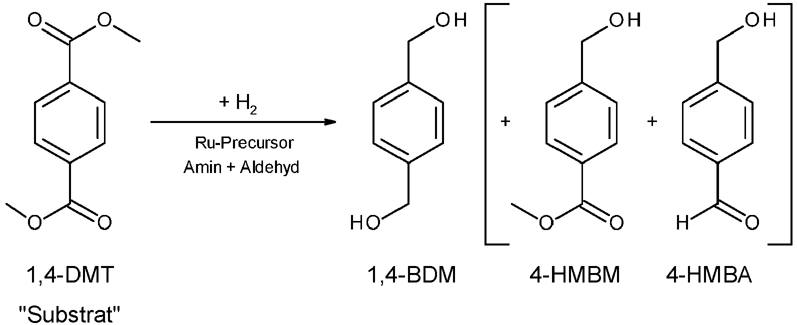

Unter Schutzgas wurden das ausgewählte Amin (gemäß Angabe im jeweiligen Beispiel) und der ausgewählte Aldehyd (gemäß Angabe im jeweiligen Beispiel) in einem 100 mL-Autoklaven vorgelegt und mit 20 mL Toluol versetzt. Der Autoklav wurde verschlossen und für 2 h auf 110°C erhitzt. Anschließend wurde auf Raumtemperatur abgekühlt und Ru-Precursor 1 (gemäß Angabe im jeweiligen Beispiel), 1,4-Dimethylterephthalsäuremethylester (gemäß Angabe im jeweiligen Beispiel) und NaOMe (gemäß Angabe im jeweiligen Beispiel) zugegeben. Es wurden erneut 20 mL Toluol zugegeben, ein Wasserstoffdruck von 6,0 MPa abs angelegt und bei 700 rpm auf 130°C erhitzt. Nach dem Erreichen von 130°C Innentemperatur wurde ein Wasserstoffdruck von 8,0 MPa abs eingestellt. Nach Ablauf der gewünschten Reaktionszeit bei 130°C wurde der Autoklav auf Raumtemperatur abgekühlt, der erhaltene Austrag eingeengt, gegebenenfalls die Ausbeute bestimmt und der Austrag per GC analysiert (Auflösen einer Probe in Dioxan). Optima FFAP Säule (30 m x 0.25 mm / 0.5 µm; 15 min bei 140°C dann mit 20°C/min auf 250°C; Durchfluss: 2.0 mL/min; Wasserstoff als Trägergas). Umsatzbestimmung mittels GC-FI.%. t_{R}(1,4-BDM) = 24.9 min; t_{R}(4-HMBM) = 23.0 min, t_{R}(4-HMBA) = 22.5 min.

### Arbeitsvorschrift 3 (isolierter Ligand / Substratscreening mit Base)

Unter Schutzgas wurden der ausgewählte Ligand (gemäß Angabe im jeweiligen Beispiel), der ausgewählte Ru-Precursor (gemäß Angabe im jeweiligen Beispiel), der ausgewählte Ester (gemäß Angabe im jeweiligen Beispiel) und KOMe (gemäß Angabe im jeweiligen Beispiel) in einem 100 mL-Autoklaven vorgelegt und mit 20 mL Toluol versetzt. Der Autoklav wurde verschlossen, ein Wasserstoffdruck von 6,0 MPa abs angelegt und bei 700 rpm auf die gewünschte Reaktionstemperatur erhitzt (gemäß Angabe im jeweiligen Beispiel). Nach Erreichen der gewünschten Reaktionstemperatur wurde ein Wasserstoffdruck von 8,0 MPa abs eingestellt. Nach Ablauf der gewünschten Reaktionszeit bei der gewünschten Reaktionstemperatur wurde der Autoklav auf Raumtemperatur abgekühlt und ein Aliquot des erhaltenen Austrags mittels GC analysiert. HP5 Säule (60 m x 0.25 mm / 1.0 µm; 5 min bei 60°C dann mit 20°C/min auf 250°C; Durchfluss: 2.0 mL/min; Helium als Trägergas). Die GC-Ausbeute wurde mittels Tetrahydropyran (THP) als internem Standard bestimmt. t_{R}(THP) = 8.4 min; t_{R}(Benzylalkohol) = 13.0 min; t_{R}(Benzoesäuremethylester) = 13.6 min.

### Arbeitsvorschrift 4 (isolierter Ligand / Substratscreening ohne Base)

Arbeitsvorschrift 4 entspricht Arbeitsvorschrift 3 mit dem Unterschied, dass die Reaktion ohne Zusatz von KOMe durchgeführt wurde.

### Arbeitsvorschrift 5 (isolierter Ligand / Substratscreening)

Arbeitsvorschrift 5 entspricht Arbeitsvorschrift 3 mit dem Unterschied, dass die Reaktion in THF anstelle von Toluol als Lösungsmittel und einem anderen Konzentrationsverhältnis durchgeführt wurde, sowie NaOMe als Base eingesetzt wurde.

Unter Schutzgas wurden der ausgewählte Ligand (gemäß Angabe im jeweiligen Beispiel), der ausgewählte Ru-Precursor (gemäß Angabe im jeweiligen Beispiel), der ausgewählte Ester (gemäß Angabe im jeweiligen Beispiel) und NaOMe (gemäß Angabe im jeweiligen Beispiel) in einem 100 mL-Autoklaven vorgelegt und mit 40 mL THF versetzt. Der Autoklav wurde verschlossen, ein Wasserstoffdruck von 6,0 MPa abs angelegt und bei 700 rpm auf die gewünschte Reaktionstemperatur erhitzt (gemäß Angabe im jeweiligen Beispiel). Nach Erreichen der gewünschten Reaktionstemperatur wurde ein Wasserstoffdruck von 8,0 MPa abs eingestellt. Nach Ablauf der gewünschten Reaktionszeit bei der gewünschten Reaktionstemperatur wurde der Autoklav auf Raumtemperatur abgekühlt und ein Aliquot des erhaltenen Austrags mittels GC analysiert. Optima FFAP Säule (30 m x 0.25 mm / 0.5 µm; 5 min bei 140°C dann mit 15°C/min auf 250°C; Durchfluss: 2.0 mL/min; Helium als Trägergas). Umsatzbestimmung mittels GC-FI.%.

### Beispiel 2

In Beispiel 2 wurde die Hydrierung von 1,4-Dimethylterephthalsäuremethylester (1,4-DMT) zu 1,4-Benzoldimethanol (1,4-BDM) in Gegenwart eines Ruthenium-Komplexes nach Arbeitsvorschrift 1 unter Einsatz verschiedener vorab synthetisierter und isolierter Liganden und verschiedener Ru-Precursor untersucht. Die Daten der Beispiele 2.1 bis 2.8 sind in Tabelle 5 dargestellt.

Unter Einsatz der Liganden L1, L3 und L4 sowie der Ru-Precursor 1, 2 und 3 wurden sehr hohe Umsätze an 1,4-DMT von bis zu > 98% sowie sehr hohe Selektivitäten zu 1,4-BDM von bis zu > 98% erreicht.

### Beispiel 3

In Beispiel 3 wurde die Hydrierung von 1,4-Dimethylterephthalsäuremethylester (1,4-DMT) zu 1,4-Benzoldimethanol (1,4-BDM) in Gegenwart eines Ruthenium-Komplexes nach Arbeitsvorschrift 2 (ohne Isolierung der Zwischenstufen) unter der Herstellung verschiedener Liganden und den Einsatz verschiedener Ru-Precursor untersucht. Die Daten der Beispiele 3.1 bis 3.5 sind in Tabelle 6 dargestellt.

Unter Einsatz der Amine 1, 2 und 3, des Aldehyds 1, aus denen sich die Liganden L1, L2 und L8 bildeten, sowie der Ru-Precursor 1, 3 und 4 wurden sehr hohe Umsätze an 1,4-DMT von bis zu > 98% sowie sehr hohe Selektivitäten zu 1,4-BDM von bis zu > 98% erreicht.

### Beispiel 4

In Beispiel 4 wurde die Hydrierung von Benzoesäuremethylester zu Benzylalkohol in Gegenwart von Ru-Komplex 1 untersucht. Ru-Komplex 1 wurde dabei entsprechend der im experimentellen Teil von P. Rigo et al., Organometallics 2007, Vol. 26, Seiten 5636-5642 beschriebenen Vorschrift mit dem Titel "Synthesis of trans-[RuCl₂(PPh₃)(b)] (1)]" hergestellt.

Unter Schutzgas wurden 36,7 µmol Ru-Komplex 1 (30 mg), 36,7 mmol Benzoesäuremethylester und 1,84 mmol KOMe in einem 100 mL-Autoklaven vorgelegt und mit 20 mL Toluol versetzt. Der Autoklav wurde verschlossen, ein Wasserstoffdruck von 6,0 MPa abs angelegt und bei 700 rpm auf 130°C erhitzt. Nach Erreichen der 130°C wurde ein Wasserstoffdruck von 8,0 MPa abs eingestellt. Nach 16 h bei 130°C wurde der Autoklav auf Raumtemperatur abgekühlt und ein Aliquot des erhaltenen Austrags mittels GC analysiert. HP5 Säule (60 m x 0.25 mm / 1.0 µm; 5 min bei 60°C dann mit 20°C/min auf 250°C; Durchfluss: 2.0 mL/min; Helium als Trägergas). Die GC-Ausbeute wurde mittels Tetrahydropyran (THP) als internem Standard bestimmt. t_{R}(THP) = 8.4 min; t_{R}(Benzylalkohol) = 13.0 min; t_{R}(Benzoesäuremethylester) = 13.6 min.

Es wurde folgendes Ergebnis erzielt:

| | |
|---|---|
| Umsatz von Benzoesäuremethylester: | >99% |
| Selektivität zu Benzylalkohol: | 99,3% |
| Selektivität zu Benzaldehyd: | 0,74% |

Auch beim Einsatz des vorab synthetisierten Ruthenium-Komplexes wurde in der Hydrierung von Benzoesäuremethylester zu Benzylalkohol ein sehr hoher Umsatz von >99% sowie eine sehr hohe Selektivität zum Benzylalkohol von > 99% erreicht.

### Beispiel 5

In Beispiel 5 wurde die Hydrierung von verschiedenen Estern (gemäß Angabe in Tabelle 7) in Gegenwart eines Ruthenium-Komplexes nach den Arbeitsvorschriften 3, 4 und 5 unter Einsatz verschiedener vorab synthetisierter und isolierter Liganden und den Ru-Precursorn 2, 3 und 5 untersucht. Tabelle 7 zeigt die Daten der Beispiele 5.1 bis 5.17.

Die Beispiele 5.1 bis 5.17 zeigen, dass das erfindungsgemäße Verfahren breit einsetzbar ist und auch beim Einsatz der verschiedensten Ester, Ru-Precursor und Liganden hohe Umsätze und hohe Selektivitäten zu den entsprechenden Alkoholen ermöglicht.

### Beispiel 6

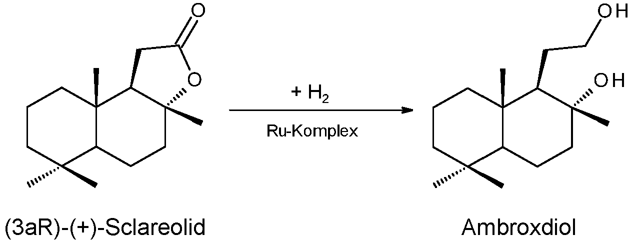

In Beispiel 6 wurde die Hydrierung von (3aR)-(+)-Sclareolid zu Ambroxdiol untersucht.

In den Beispielen 6.1 und 6.2 wurden unter Schutzgas Ru-Komplex 1 (gemäß Angabe in Tabelle 8), (3aR)-(+)-Sclareolid (gemäß Angabe in Tabelle 8) und NaOMe (gemäß Angabe in Tabelle 8) in einem 100 mL-Autoklaven vorgelegt und mit 40 mL Tetrahydrofuran versetzt. Der Autoklav wurde verschlossen, ein Wasserstoffdruck von 6,0 MPa abs angelegt und bei 700 rpm auf die gewünschte Reaktionstemperatur erhitzt (gemäß Angabe in Tabelle 8). Nach Erreichen der gewünschten Reaktionstemperatur wurde ein Wasserstoffdruck von 8,0 MPa abs eingestellt. Nach Ablauf der gewünschten Reaktionszeit bei der gewünschten Reaktionstemperatur wurde der Autoklav auf Raumtemperatur abgekühlt und die erhaltene Lösung mittels GC analysiert: Optima FFAP column (30 m x 0.25 mm / 0.5 µm; 15 min bei 140°C dann mit 20°C/min auf 250 °C; Durchfluss: 2,0 ml/min; Helium als Trägergas). t_{R}(Sclareolid) = 29,4 min; t_{R}(Ambroxdiol) = 32,5 min.

In Beispiel 6.3 wurde die Hydrierung von (3aR)-(+)-Sclareolid zu Ambroxdiol in Gegenwart eines Ruthenium-Komplexes untersucht. Die Durchführung erfolgte wie in den Beispielen 6.1 und 6.2, allerdings wurde im Unterschied zu den Beispielen 6.1 und 6.2 anstelle des Ru-Komplexes 1 der Ligand L3 und der Ru-Precursor 3 eingesetzt.

Tabelle 8 zeigt die Daten der Beispiele 6.1 bis 6.3.

### Beispiel 7

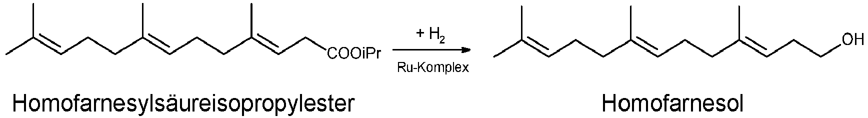

In Beispiel 7 wurde die Hydrierung von Homofarnesylsäureisopropylester zu Homofarnesol untersucht.

Unter Schutzgas wurde Ru-Precursor 5 (gemäß Angabe in Tabelle 9) und Ligand L3 (gemäß Angabe in Tabelle 9) in einem 100 mL-Autoklaven vorgelegt und mit 30 mL Methanol versetzt. Der Autoklav wurde verschlossen, ein Wasserstoffdruck von 5,0 MPa abs angelegt und bei 700 rpm für 1.5 h auf 60°C erhitzt. Dann wird nochmals kurz entspannt und unter inerter Atmosphäre NaOMe und Homofarnesylsäureisopropylester gelöst in 10 mL Methanol zugegeben (gemäß Angabe in Tabelle 9). Der Wasserstoffdruck wird anschließend auf 5,0 MPa eingestellt und es wird bei 700 rpm auf die gewünschte Reaktionstemperatur erhitzt (gemäß Angabe in Tabelle 9). Nach Erreichen der gewünschten Reaktionstemperatur wurde ein Wasserstoffdruck von 8,0 MPa abs eingestellt. Nach Ablauf der angegebenen Reaktionszeit wurde der Autoklav auf Raumtemperatur abgekühlt und die erhaltene Lösung mittels GC analysiert: VF-23ms column (60 m x 0.25 mm / 0.25 µm; 5 min bei 50°C dann mit 5°C/min auf 250°C; Durchfluss: 1,0 ml/min; Helium als Trägergas). t_{R}(Homofarnesylsäureisopropylester) = 34,3 min; t_{R}(Homofarnesol, sum of 4 isomers) = 35.1, 35.3, 35.7, 35.8 min.

Tabelle 9 zeigt die Daten von Beispiel 7.

### Beispiel 8

Beispiel 8 zeigt die Wiederverwendung des Katalysators nach der destillativen Entfernung des Produktes aus der ersten Hydrierung (Katalysatorrecycling)

Unter Schutzgas wurden 36,7 µmol L3, 12,2 µmol Ru-Precursor 5 und 36,7 mmol Benzoesäuremethylester in einem 100 mL-Autoklaven vorgelegt und mit 20 mL Benzylalkohol versetzt. Der Autoklav wurde verschlossen, ein Wasserstoffdruck von 7,0 MPa abs angelegt und bei 700 rpm auf 130°C erhitzt. Nach Erreichen der Reaktionstemperatur wurde ein Wasserstoffdruck von 8,0 MPa abs eingestellt. Nach Ablauf von 16 Stunden Reaktionszeit bei 130°C wurde der Autoklav auf Raumtemperatur abgekühlt und ein Aliquot des erhaltenen Austrags mittels GC analysiert. HP5 Säule (60 m x 0.25 mm / 1.0 µm; 5 min bei 60°C dann mit 20°C/min auf 250°C; Durchfluss: 2.0 mL/min; Helium als Trägergas). Die GC-Ausbeute wurde mittels Tetrahydropyran (THP) als internem Standard bestimmt. t_{R}(THP) = 8.4 min; t_{R}(Benzylalkohol) = 13.0 min; t_{R}(Benzoesäuremethylester) = 13.6 min. Umsatz 99,3%; Selektivität 95% Benzylalkohol.

Der erhaltene Austrag wurde im Vakuum aufkonzentriert und anschließend wieder mit Benzylalkohol auf 20 mL Gesamtvolumen verdünnt. Die katalysatorhaltige Reaktionslösung wurde unter Schutzgas wieder in den Autoklaven überführt und es wurden erneut 36,7 mmol Benzoesäuremethylester zugegeben. Es wurde ein Wasserstoffdruck von 7,0 MPa abs angelegt und bei 700 rpm auf 130°C erhitzt. Nach Erreichen der Reaktionstemperatur wurde ein Wasserstoffdruck von 8,0 MPa abs eingestellt. Nach Ablauf von 16 Stunden Reaktionszeit bei 130°C wurde der Autoklav auf Raumtemperatur abgekühlt und ein Aliquot des erhaltenen Austrags mittels GC (Methode wie zuvor beschrieben) analysiert. Umsatz 97,8%; Selektivität 96% Benzylalkohol.

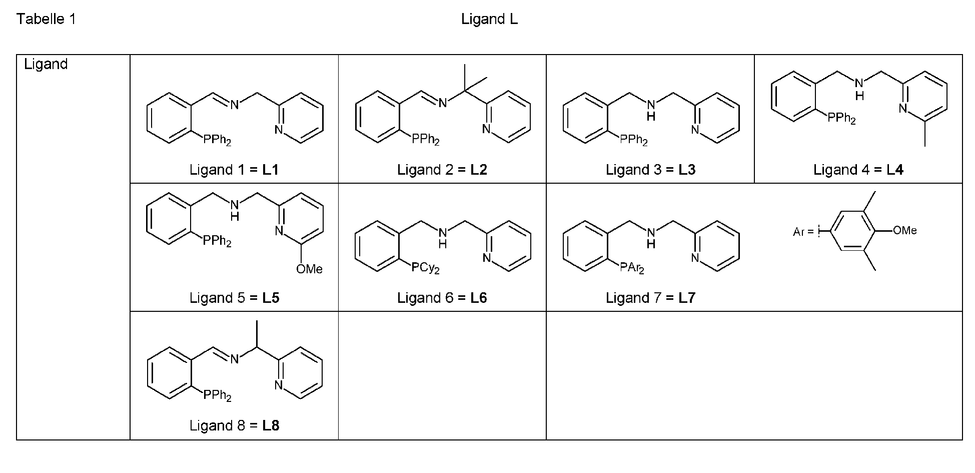

**Tabelle 2: Liganden-Precursor**

| | | | | |
|---|---|---|---|---|
| Amin | 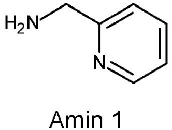 | 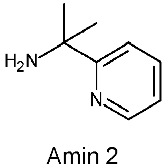 | 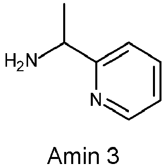 | |
| | 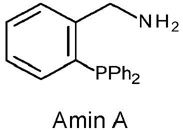 | | | |
| Aldehyd | 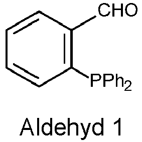 | 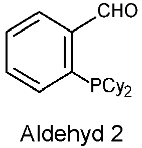 | 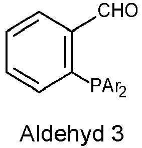 | 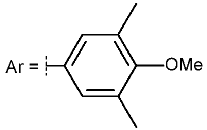 |
| | 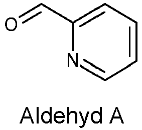 | 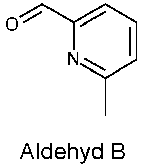 | 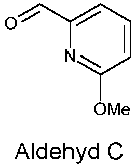 | |

**Tabelle 3: Rutheniumkomplex-Precursor**

| | | | | |
|---|---|---|---|---|
| Ru-Precursor | [Ru(p-cymol)Cl₂]₂ p-cymol = 4-Isopropyltoluol | Ru(Cl)₂(PPh₃)₃ | Ru(acac)₃ acac = Acetylacetonat | Ru(COD)(methylallyl)₂ COD = 1,5-Cyclooctadien |
| | Ru-Precursor 1 | Ru-Precursor 2 | Ru-Precursor 3 | Ru-Precursor 4 |
| | Ru₃(CO)₁₂ | | | |
| | Ru-Precursor 5 | | | |

**Tabelle 4: Ru-Komplex**

| | | |
|---|---|---|
| Ru-Komplex | 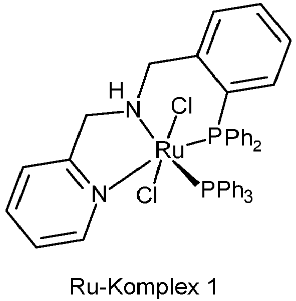 | |

**Tabelle 5 (Teil 1): Daten zu den Beispielen 2.1 bis 2.5 nach Arbeitsvorschrift 1**

| Bsp. | Einsatzmengen | Reaktionsbedingungen | Molverhältnis Ligand/Ru [mol/mol] | Molverhältnis ^{a)} Substrat/Ru [mol/mol] | Umsatz ^{b)} von 1,4-DMT | Selektivität ^{c)} |
|---|---|---|---|---|---|---|
| 2.1 | 0,051 mmol L1 aus Beispiel 1.1 | 130°C | 1,0 | 1000 | > 98% | > 98% zu 1,4-BDM |
| | 0,026 mmol Ru-Precursor 1 | | | | | |
| | 51,5 mmol 1,4-DMT (Substrat) | 16 h | | | | |
| | 2,5 mmol NaOMe | | | | | |
| 2.2 | 0,051 mmol L3 aus Beispiel 1.2 | 130°C | 1,0 | 1000 | > 98% | > 98% zu 1,4-BDM |
| | 0,026 mmol Ru-Precursor 1 | | | | | |
| | 51,5 mmol 1,4-DMT (Substrat) | 16 h | | | | |
| | 2,5 mmol NaOMe | | | | | |
| 2.3 | 0,051 mmol L4 aus Beispiel 1.3 | 130°C | 1,0 | 1000 | > 98% | 97% zu 1,4-BDM |
| | 0,026 mmol Ru-Precursor 1 | | | | | |
| | 51,5 mmol 1,4-DMT (Substrat) | 16 h | | | | |
| | 2,5 mmol NaOMe | | | | | |
| 2.4 | 0,021 mmol L3 aus Beispiel 1.2 | 130°C | 2,0 | 5000 | > 98% | > 98% zu 1,4-BDM |
| | 0,005 mmol Ru-Precursor 1 | | | | | |
| | 51,5 mmol 1,4-DMT (Substrat) | 60 h | | | | |
| | 2,5 mmol NaOMe | | | | | |
| 2.5 | 0,010 mmol L3 aus Beispiel 1.2 | 130°C | 1,0 | 5000 | > 98% | > 98% zu 1,4-BDM |
| | 0,005 mmol Ru-Precursor 1 | | | | | |
| | 51,5 mmol 1,4-DMT (Substrat) | 60 h | | | | |
| | 2,5 mmol NaOMe | | | | | |

| | | | | | | |
|---|---|---|---|---|---|---|
| a) gerundete Werte, b) Umsatz nach Reaktionsende, c) Selektivität nach Reaktionsende | | | | | | |

**Tabelle 5 (Teil 2): Daten zu den Beispielen 2.6 bis 2.8 nach Arbeitsvorschrift 1**

| Bsp. | Einsatzmengen | Reaktionsbedingungen | Molverhältnis Ligand/Ru [mol/mol] | Molverhältnis ^{a)} Substrat/Ru [mol/mol] | Umsatz ^{b)} von 1,4-DMT | Selektivität ^{c)} |
|---|---|---|---|---|---|---|
| 2.6 | 0,051 mmol L3 aus Beispiel 1.2 | 130°C | 1,0 | 1000 | > 98% | > 98% zu 1,4-BDM |
| | 0,051 mmol Ru-Precursor 2 | | | | | |
| | 51,5 mmol 1,4-DMT (Substrat) | 16 h | | | | |
| | 2,5 mmol NaOMe | | | | | |
| 2.7 | 0,051 mmol L3 aus Beispiel 1.2 | 130°C | 1,0 | 1000 | > 98% | > 98% zu 1,4-BDM |
| | 0,051 mmol Ru-Precursor 3 | | | | | |
| | 51,5 mmol 1,4-DMT (Substrat) | 16 h | | | | |
| | 2,5 mmol NaOMe | | | | | |
| 2.8 | 0,051 mmol L3 aus Beispiel 1.2 | 130°C | 1,0 | 1000 | > 98% | > 98% zu 1,4-BDM 1% zu 4-HMBA |
| | 0,026 mmol Ru-Precursor 1 | | | | | |
| | 51,5 mmol 1,4-DMT (Substrat) | 16 h | | | | |
| | 2,5 mmol KOMe ^{d)} | | | | | |

| | | | | | | |
|---|---|---|---|---|---|---|
| a) gerundete Werte, b) Umsatz nach Reaktionsende, c) Selektivität nach Reaktionsende d) mit KOMe anstatt von NaOMe | | | | | | |

**Tabelle 6 (Teil 1): Daten zu den Beispielen 3.1 bis 3.4 nach Arbeitsvorschrift 2**

| Bsp. | Einsatzmengen | Reaktionsbedingungen | Molverhältnis Ligand/Ru [mol/mol] | Molverhältnis ^{a)} Substrat/Ru [mol/mol] | Umsatz ^{b)} von 1,4-DMT | Selektivität ^{c)} |
|---|---|---|---|---|---|---|
| 3.1 | 0,064 mmol Aldehyd 1 | 130°C | 1,28 | 1000 | > 98% | > 98% zu 1,4-BDM |
| | 0,064 mmol Amin 1 | | | | | |
| | 0,025 mmol Ru-Precursor 1 | | | | | |
| | | 16 h | | | | |
| | 51,5 mmol 1,4-DMT (Substrat) | | | | | |
| | 2,5 mmol NaOMe | | | | | |
| 3.2 | 0,064 mmol Aldehyd 1 | 130°C | 1,28 | 1000 | > 98% | 44% zu 1,4-BDM |
| | 0,064 mmol Amin 1 | | | | | |
| | 0,05 mmol Ru-Precursor 3 | | | | | |
| | | 16 h | | | | 53% zu 4-HMBM |
| | 51,5 mmol 1,4-DMT (Substrat) | | | | | |
| | 2,5 mmol NaOMe | | | | | |
| 3.3 | 0,064 mmol Aldehyd 1 | 130°C | 1,28 | 1000 | > 98% | 97% zu 1,4-BDM |
| | 0,064 mmol Amin 1 | | | | | |
| | 0,05 mmol Ru-Precursor 4 | | | | | |
| | | 16 h | | | | |
| | 51,5 mmol 1,4-DMT (Substrat) | | | | | |
| | 2,5 mmol NaOMe | | | | | |
| 3.4 | 0,064 mmol Aldehyd 1 | 130°C | 1,28 | 1000 | > 98% | > 98% zu 1,4-BDM |
| | 0,064 mmol Amin 2 | | | | | |
| | 0,025 mmol Ru-Precursor 1 | | | | | |
| | | 16 h | | | | |
| | 51,5 mmol 1,4-DMT (Substrat) | | | | | |
| | 2,5 mmol NaOMe | | | | | |

| | | | | | | |
|---|---|---|---|---|---|---|
| a) gerundete Werte, b) Umsatz nach Reaktionsende, c) Selektivität nach Reaktionsende | | | | | | |

**Tabelle 6 (Teil 2): Daten zu Beispiel 3.5 nach Arbeitsvorschrift 2.**

| Bsp. | Einsatzmengen | Reaktionsbedingungen | Molverhältnis Ligand/Ru [mol/mol] | Molverhältnis ^{a)} Substrat/Ru [mol/mol] | Umsatz ^{b)} von 1,4-DMT | Selektivität ^{c)} |
|---|---|---|---|---|---|---|
| 3.5 | 0,064 mmol Aldehyd 1 | 130°C | 1,28 | 1000 | > 98% | 85% zu 1,4-BDM |
| | 0,064 mmol Amin 3 | | | | | |
| | 0,025 mmol Ru-Precursor 1 | | | | | |
| | | 16 h | | | | 14% zu 4-HMBM |
| | 51,5 mmol 1,4-DMT (Substrat) | | | | | |
| | 2,5 mmol NaOMe | | | | | |

| | | | | | | |
|---|---|---|---|---|---|---|
| a) gerundete Werte, b) Umsatz nach Reaktionsende, c) Selektivität nach Reaktionsende | | | | | | |

**Tabelle 7 (Teil 1): Daten zu den Beispielen 5.1 bis 5.4 nach Arbeitsvorschrift 3**

| Bsp. | Einsatzmengen | Substrat | Reaktionsbedingungen | Alkohol | Umsatz ^{a)} des Substrats | Selektivität ^{b)} zum Alkohol |
|---|---|---|---|---|---|---|
| 5.1 | Arbeitsvorschrift 3 | 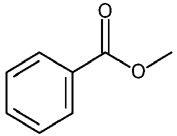 Benzoesäuremethylester | 130°C | Benzylalkohol | 99% | 99% |
| | 36,7 µmol L3 aus Beispiel 1.2 | | | | | |
| | 36,7 µmol Ru-Precursor 2 | | | | | |
| | | | 16 h | | | |
| | 36,7 mmol Substrat | | | | | |
| | 1,84 mmol KOMe | | | | | |
| 5.2 | Arbeitsvorschrift 3 | 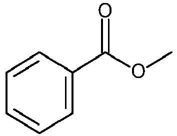 Benzoesäuremethylester | 130 °C | Benzylalkohol | 99 | 97 |
| | 183,5 µmol L4 aus Beispiel 1.3 | | | | | |
| | 183,5 µmol Ru-Precursor 2 | | | | | |
| | | | 16 h | | | |
| | 36,7 mmol Substrat | | | | | |
| | 3,68 mmol KOMe | | | | | |
| 5.3 | Arbeitsvorschrift 3 | 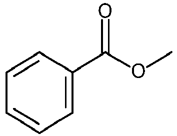 Benzoesäuremethylester | 130 °C | Benzylalkohol | 99% | 98% |
| | 183,5 µmol L5 aus Beispiel 1.4 | | | | | |
| | 183,5 µmol Ru-Precursor 2 | | | | | |
| | | | 16 h | | | |
| | 36,7 mmol Substrat | | | | | |
| | 3,68 mmol KOMe | | | | | |
| 5.4 | Arbeitsvorschrift 3 | 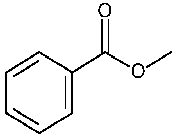 Benzoesäuremethylester | 130 °C | Benzylalkohol | 99% | 97% |
| | 183,5 µmol L6 aus Beispiel 1.5 | | | | | |
| | 183,5 µmol Ru-Precursor 2 | | | | | |
| | | | 16 h | | | |
| | 36,7 mmol Substrat | | | | | |
| | 3,68 mmol KOMe | | | | | |

| | | | | | | |
|---|---|---|---|---|---|---|
| a) Umsatz nach Reaktionsende, b) Selektivität nach Reaktionsende | | | | | | |

**Tabelle 7 (Teil 2): Daten zu den Beispielen 5.5 bis 5.8 nach Arbeitsvorschrift 3**

| Bsp. | Einsatzmengen | Substrat | Reaktionsbedingungen | Alkohol | Umsatz ^{a)} des Substrats | Selektivität ^{b)} zum Alkohol |
|---|---|---|---|---|---|---|
| 5.5 | Arbeitsvorschrift 3 | 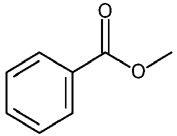 | 130°C | Benzylalkohol | 99% | 97% |
| | 183,5 µmol L7 aus Beispiel 1.6 | | | | | |
| | 183,5 µmol Ru-Precursor 2 | | | | | |
| | 36,7 mmol Substrat | | 16 h | | | |
| | 3,68 mmol KOMe | Benzoesäuremethylester | | | | |
| 5.6 | Arbeitsvorschrift 3 | 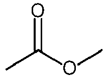 | 130°C | Ethanol | 99% | 99% |
| | 36,7 µmol L3 aus Beispiel 1.2 | | | | | |
| | 36,7 µmol Ru-Precursor 2 | | | | | |
| | 36,7 µmol Substrat | | 16 h | | | |
| | 1,84 mmol KOMe | Essigsäuremethylester | | | | |
| 5.7 | Arbeitsvorschrift 3 | 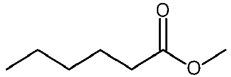 | 130°C | n-Hexanol | 99% | 97% |
| | 73,4 µmol L3 aus Beispiel 1.2 | | | | | |
| | 73,4 µmol Ru-Precursor 2 | | | | | |
| | 36,7 mmol Substrat | Hexansäuremethylester | 16 h | | | |
| | 1,84 mmol KOMe | | | | | |
| 5.8 | Arbeitsvorschrift 3 | 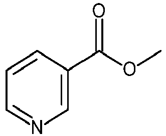 | 130°C | Nikotinylalkohol | 94% | 94% |
| | 36,7 µmol L3 aus Beispiel 1.2 | | | | | |
| | 36,7 µmol Ru-Precursor 2 | | | | | |
| | 36,7 mmol Substrat | | 16 h | | | |
| | 1,84 mmol KOMe | Nikotinsäuremethylester | | | | |

| | | | | | | |
|---|---|---|---|---|---|---|
| a) Umsatz nach Reaktionsende, b) Selektivität nach Reaktionsende | | | | | | |

**Tabelle 7 (Teil 3): Daten zu den Beispielen 5.9 bis 5.11 nach Arbeitsvorschriften 3 und 4**

| Bsp. | Einsatzmengen | Substrat | Reaktionsbedingungen | Alkohol | Umsatz ^{a)} des Substrats | Selektivität ^{b)} zum Alkohol |
|---|---|---|---|---|---|---|
| 5.9 | Arbeitsvorschrift 3 | 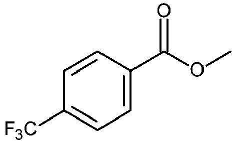 | 130°C | 4-(Trifluoromethyl)phenyl) methanol | 99% | 99% |
| | 183,5 µmol L3 aus Beispiel 1.2 | | | | | |
| | 183,5 µmol Ru-Precursor 2 | | | | | |
| | 36,7 µmol Substrat | | 16 h | | | |
| | 3,68 mmol KOMe | 4-(Trifluoromethyl) benzoesäuremethylester | | | | |
| 5.10 | Arbeitsvorschrift 3 | 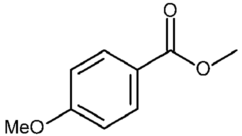 | 130°C | (4-Methoxyphenyl) methanol | 99% | 99% |
| | 183,5 µmol L3 aus Beispiel 1.2 | | | | | |
| | 183,5 µmol Ru-Precursor 2 | | | | | |
| | 36,7 µmol Substrat | | 16 h | | | |
| | 3,68 mmol KOMe | 4-(Methoxy) benzoesäuremethylester | | | | |
| 5.11 | Arbeitsvorschrift 4 | 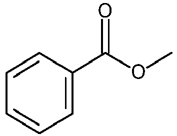 | 130°C | Benzylalkohol | 99% | 98% |
| | 36,7 µmol L3 aus Beispiel 1.2 | | | | | |
| | 12,2 µmol Ru-Precursor 5 | | 16 h | | | |
| | 36,7 mmol Substrat | Benzoesäuremethylester | | | | |

| | | | | | | |
|---|---|---|---|---|---|---|
| a) Umsatz nach Reaktionsende, b) Selektivität nach Reaktionsende | | | | | | |

**Tabelle 7 (Teil 4): Daten zu den Beispielen 5.12 bis 5.14 nach Arbeitsvorschrift 4**

| Bsp. | Einsatzmengen | Substrat | Reaktionsbedingungen | Alkohol | Umsatz ^{a)} des Substrats | Selektivität ^{b)} zum Alkohol |
|---|---|---|---|---|---|---|
| 5.12 | Arbeitsvorschrift 4 | 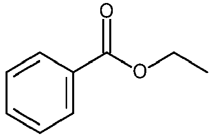 | 130°C | Benzylalkohol | 97% | 99% |
| | 46,6 µmol L3 aus Beispiel 1.2 | | | | | |
| | 46,6 µmol Ru-Precursor 3 | | 16 h | | | |
| | 33 mmol Substrat | Benzoesäureethylester | | | | |
| 5.13 | Arbeitsvorschrift 4 | 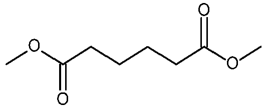 | 130°C | 1,6-Hexandiol | 99% | 99% |
| | 80,4 µmol L3 aus Beispiel 1.2 | | | | | |
| | 26,8 µmol Ru-Precursor 5 | | 16 h | | | |
| | 28,7 mmol Substrat | Dimethyladipat | | | | |
| 5.14 | Arbeitsvorschrift 4 | 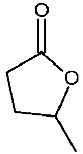 | 130°C | 1,4-Pentandiol | 97% | 99% |
| | 140 µmol L3 aus Beispiel 1.2 | | | | | |
| | 46,6 µmol Ru-Precursor 5 | | 16 h | | | |
| | 49,9 mmol Substrat | γ-Valerolacton | | | | |

| | | | | | | |
|---|---|---|---|---|---|---|
| a) Umsatz nach Reaktionsende, b) Selektivität nach Reaktionsende | | | | | | |

**Tabelle 7 (Teil 5): Daten zu den Beispielen 5.15 bis 5.17 nach Arbeitsvorschrift 5**

| Bsp. | Einsatzmengen | Substrat | Reaktionsbedingungen | Alkohol | Umsatz a) des Substrats | Selektivität ^{b)} zum Alkohol |
|---|---|---|---|---|---|---|
| 5.15 | Arbeitsvorschrift 5 | 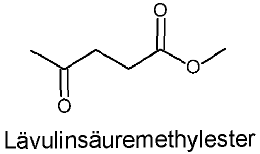 | 130°C | 1,4-Pentandiol | >99% | >99% |
| | 38 µmol L3 aus Beispiel 1.2 | | | | | |
| | 19 µmol Ru-Precursor 1 | | 16 h | | | |
| | 38,4 mmol Substrat | | | | | |
| | 1,45 mmol NaOMe | | | | | |
| 5.16 | Arbeitsvorschrift 5 | 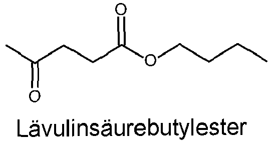 | 130°C | 1,4-Pentandiol | >99% | 87% 1,4-Pentandiol |
| | 29 µmol L3 aus Beispiel 1.2 | | | | | |
| | 15 µmol Ru-Precursor 1 | | 16 h | | | 13% γ-Valerolacton |
| | 29 mmol Substrat | | | | | |
| | 1,45 mmol NaOMe | | | | | |
| 5.17^{c)} | Arbeitsvorschrift 5 | 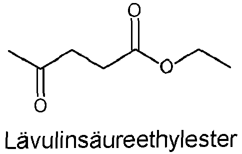 | 130°C | 1,4-Pentandiol | >99% | >99% |
| | 35 µmol L3 aus Beispiel 1.2 | | | | | |
| | 12 µmol Ru-Precursor 5 | | 16 h | | | |
| | 34,7 mmol Substrat | | | | | |
| | 1,73 mmol NaOMe | | | | | |

| | | | | | | |
|---|---|---|---|---|---|---|
| a) Umsatz nach Reaktionsende, b) Selektivität nach Reaktionsende c) Ru-Precursor 5 und L3 wurden zunächst 90 min bei 60°C und 50 bar H₂ in THF (20 mL) gerührt und anschließend das Substrat in THF (20 mL) zugegeben. Die weitere Durchführung erfolgte dann wie in Arbeitsvorschrift 5 beschrieben. | | | | | | |

**Tabelle 8: Daten zu den Beispielen 6.1 bis 6.3**

| Bsp. | Einsatzmengen | Reaktionsbedingungen | Molverhältnis ^{a)} Substrat/Ru [mol/mol] | Umsatz ^{b)} von Sclareolid | Selektivität ^{c)} (Ambroxdiol) |
|---|---|---|---|---|---|
| 6.1 | 20 µmol Ru-Komplex 1 | 130°C | 1000 | 96% | 90% |
| | 20 mmol (3aR)-(+)-Sclareolid (Substrat) | 16 h | | | |
| | 1 mmol NaOMe | | | | |
| 6.2 | 4 µmol Ru-Komplex 1 | 135°C | 5000 | 97% | 88% |
| | 20 mmol (3aR)-(+)-Sclareolid (Substrat) | | | | |
| | 1 mmol NaOMe | 60 h | | | |
| 6.3 | 8 µmol L3 aus Beispiel 1.2 | 135°C | 5000 | 84% | 85% |
| | 8 µmol Ru-Precursor 3 | | | | |
| | 40 mmol (3aR)-(+)-Sclareolid (Substrat) | 60 h | | | |
| | 2 mmol NaOMe | | | | |

| | | | | | |
|---|---|---|---|---|---|
| a) gerundete Werte, b) Umsatz nach Reaktionsende, c) Selektivität nach Reaktionsende | | | | | |

**Tabelle 9: Daten zu Beispiel 7**

| Bsp. | Einsatzmengen | Reaktionsbedingungen | Molverhältnis ^{a)} Substrat/Ru [mol/mol] | Umsatz ^{b)} des Substrats | Selektivität^{c)} zum Alkohol |
|---|---|---|---|---|---|
| 7 | 200 µmol L3 aus Beispiel 1.2 | 100°C | 200 | 98% | 94% |
| | 66 µmol Ru-Precursor 5 | | | | |
| | 40 mmol Homofarnesylsäureisopropylester | 10 h | | | |
| | 2 mmol NaOMe | | | | |

| | | | | | |
|---|---|---|---|---|---|
| a) gerundete Werte, b) Umsatz nach Reaktionsende, c) Selektivität nach Reaktionsende | | | | | |

## Patentansprüche

1. Verfahren zur Hydrierung eines Esters mit molekularem Wasserstoff zu den korrespondierenden Alkoholen bei einer Temperatur von 50 bis 200°C und einem Druck von 0,1 bis 20 MPa abs in Gegenwart eines fünffach oder sechsfach koordinierten Ruthenium-Komplexes (I), wobei der Ruthenium-Komplex auch zu einem Dimer verbrückt sein kann, **dadurch gekennzeichnet, dass** der Ruthenium-Komplex einen dreizähnigen Liganden L mit der allgemeinen Formel (II) 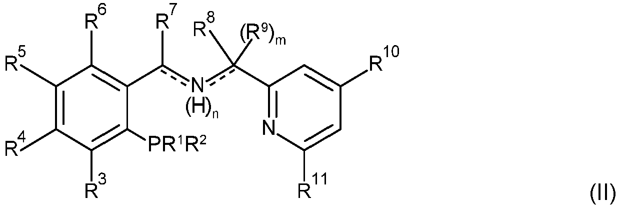 enthält, wobei
R¹, R² unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
R³, R⁴, R⁵, R⁶, R¹⁰, R¹¹ unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
R⁷, R⁸, R⁹ unabhängig voneinander für Wasserstoff, lineares C₁ bis C₄-Alkyl oder verzweigtes C₃ bis C₄-Alkyl stehen,
n, m unabhängig voneinander 0 oder 1 sind, und
die durchgezogen-gestrichelten Doppellinien für eine Einfach- oder Doppelbindung stehen, mit der Maßgabe, dass
| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung darstellt, wobei im Falle einer Doppelbindung auf der dem Phenylring zugewandten Seite m = 1 ist, im Falle einer Doppelbindung auf der dem Pyridylring zugewandten Seite m = 0 ist, oder beide durchgezogen-gestrichelten Doppellinien eine Einfachbindung darstellen und m gleich 1 ist. |

2. Verfahren nach Anspruch 1, **dadurch gekennzeichnet, dass** man einen Liganden L (II) einsetzt, bei dem
(i) n und m jeweils 1 ist und die beiden durchgezogen-gestrichelten Doppellinien eine Einfachbindung darstellen, oder
(ii) n gleich 0 und m gleich 1 ist und die dem Phenylring zugewandte durchgezogen-gestrichelte Doppellinie eine Doppelbindung und die dem Pyridylring zugewandte durchgezogen-gestrichelte Doppellinie eine Einfachbindung darstellt,
und
beide Reste R¹ und R² für Phenyl, p-Tolyl, 3,5-Dimethyl-4-methoxyphenyl, iso-Butyl oder Cyclohexyl stehen,
die Reste R³, R⁴ und R⁶ für Wasserstoff stehen,
die Reste R⁵ und R¹⁰ für Wasserstoff, Methyl oder tert.-Butyl stehen,
der Rest R¹¹ für Wasserstoff, Methyl oder Methoxy steht, und
die Reste R⁷, R⁸ und R⁹ für Wasserstoff oder Methyl stehen.

3. Verfahren nach den Ansprüchen 1 bis 2, **dadurch gekennzeichnet, dass** der Ruthenium-Komplex (I) Ruthenium in der Oxidationsstufe +2 oder +3 enthält und die allgemeine Formel (IA)
[Ru(L)XₐY_{b}]ₚ Z_{(p · c)} (IA)
aufweist, wobei
X jeweils unabhängig voneinander für einen neutralen monodentaten Liganden steht, wobei zwei Liganden X auch zu einem neutralen bidentaten Liganden verbunden sein können,
Y jeweils unabhängig voneinander für einen anionischen monodentaten Liganden mit der Ladung "-1" steht,
wobei Y und X zusammen auch für einen anionischen bidentaten Liganden mit der Ladung "-1" stehen kann,
Z jeweils unabhängig voneinander für ein nicht-koordinierendes Anion mit der Ladung "-1" steht, wobei zwei Liganden Z auch zu einem nicht-koordinierenden Anion mit der Ladung "-2" verbunden sein können,
a, b und c jeweils unabhängig voneinander für 0, 1, 2 oder 3 stehen, und
p für 1 oder 2 steht,
mit den Maßgaben, dass
a + b + c gleich 1, 2, 3, 4, 5 oder 6 ist, und
b und c derart bestimmt sind, dass der Ruthenium-Komplex (IA) die Gesamtladung "0" aufweist.

4. Verfahren nach den Ansprüchen 1 bis 3, **dadurch gekennzeichnet, dass** man den Ruthenium-Komplex (I) durch Umsetzung von Ligand (II) mit einem Ru-Precursorkomplex (IV) erhält.

5. Verfahren nach den Ansprüchen 1 bis 4, **dadurch gekennzeichnet, dass** man als Ester einen Ester der allgemeinen Formel (III) 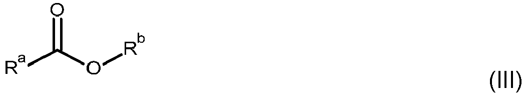 einsetzt, bei dem die Reste R^{a} und R^{b} jeweils unabhängig voneinander für einen Kohlenstoff enthaltenden organischen, linearen oder verzweigten, nichtcyclischen oder cyclischen, gesättigten oder ungesättigten, aliphatischen, aromatischen oder araliphatischen, unsubstituierten oder durch Heteroatome oder funktionelle Gruppen unterbrochenen oder substituierten Rest mit einer Molmasse von 15 bis 10000 g/mol stehen, wobei beide Reste R^{a} und R^{b} auch miteinander verbunden sein können.

6. Verfahren nach den Ansprüchen 1 bis 5, **dadurch gekennzeichnet, dass** man den Ruthenium-Komplex (I) in-situ aus einem Ru-Precursorkomplex (IV) und Ligand L (II) bildet.

7. Verfahren nach den Ansprüchen 1 bis 5, **dadurch gekennzeichnet, dass** man den Ruthenium-Komplex (I) in-situ durch Umsetzung
(a) eines Aldehyds oder Ketons der allgemeinen Formel (Va) 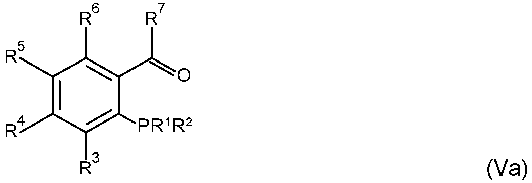 mit einem Amin der allgemeinen Formel (Vb) 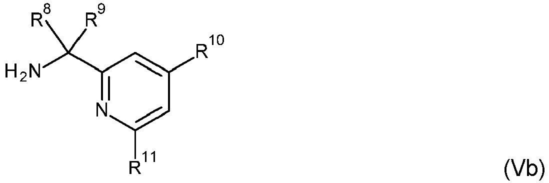 und/oder
(b) eines Amins der allgemeinen Formel (VIa) 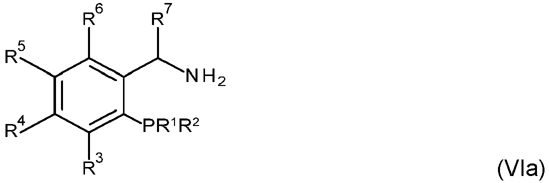 mit einem Aldehyd oder einem Keton der allgemeinen Formel (Vlb) 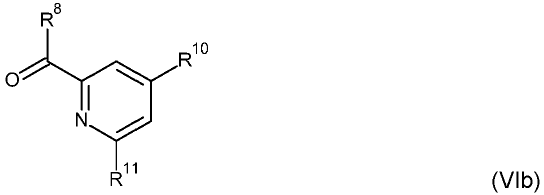 zum Liganden L (II), wobei die Reste R¹ bis R¹¹ jeweils die zuvor definierte Bedeutung haben, und nachfolgender Umsetzung des gebildeten Liganden L (II) ohne dessen Isolierung oder Aufreinigung mit einem Ru-Precursorkomplex (IV) bildet.

8. Verfahren nach den Ansprüchen 1 bis 7, **dadurch gekennzeichnet, dass** man ein Molverhältnis zwischen dem Ester und dem Ruthenium-Komplex (I) von 1 bis 100000 einsetzt.

9. Verfahren nach den Ansprüchen 1 bis 8, **dadurch gekennzeichnet, dass** man die Hydrierung in Gegenwart einer Base durchführt.

10. Verfahren nach Anspruch 9, **dadurch gekennzeichnet, dass** man als Base Alkoholate oder Amide einsetzt.

## Claims

1. A method for hydrogenating an ester with molecular hydrogen to give the corresponding alcohols at a temperature of 50 to 200°C and a pressure of 0.1 to 20 MPa abs in the presence of a five-fold or six-fold coordinated ruthenium complex (I), where the ruthenium complex can also be bridged to form a dimer, wherein the ruthenium complex comprises a tridentate ligand L of the general formula (II) 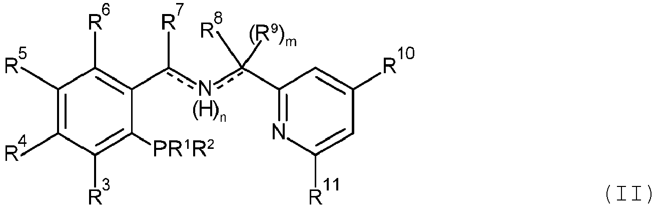 where
R¹, R² are each independently an aliphatic hydrocarbon radical having 1 to 8 carbon atoms, an aromatic hydrocarbon radical having 6 or 10 carbon atoms or an araliphatic hydrocarbon radical having 7 to 12 carbon atoms, where the hydrocarbon radicals specified are unsubstituted or substituted by 1 to 3 methoxy, thiomethoxy or dimethylamino groups, and the two radicals R¹ and R² may also be bonded to each other to form a 5-to 10-membered ring including the phosphorus atom,
R³, R⁴, R⁵, R⁶, R¹⁰, R¹¹ are each independently hydrogen, linear C₁ to C₄-alkyl, branched C₃ to C₄-alkyl, methoxy, hydroxyl, trifluoromethyl, nitrile or dialkylamino each independently having 1 to 4 carbon atoms per alkyl group, R⁷, R⁸, R⁹ are each independently hydrogen, linear C₁ to C₄-alkyl or branched C₃ to C₄-alkyl,
n, m are each independently 0 or 1, and
the solid-dashed double lines are a single or double bond, with the proviso that
| | |
|---|---|
| in the case of n = 1, | both solid-dashed double lines represent a single bond and m is 1, and |
| in the case of n = 0, | one solid-dashed double line represents a single bond and the other solid-dashed double line represents a double bond, where in the case of a double bond on the side facing the phenyl ring m = 1, in the case of a double bond on the side facing the pyridyl ring m = 0, or both solid-dashed double lines represent a single bond and m equals 1. |

2. The method according to claim 1, wherein a ligand L (II) is used in which
(i) n and m are in each case 1 and the two solid-dashed double lines represent a single bond, or
(ii) n is 0 and m is 1 and the solid-dashed double line facing the phenyl ring represents a double bond and the solid-dashed double line facing the pyridyl ring represents a single bond,
and
both radicals R¹ and R² are phenyl, p-tolyl, 3, 5-dimethyl-4-methoxyphenyl, isobutyl or cyclohexyl,
the radicals R³, R⁴ and R⁶ are hydrogen,
the radicals R⁵ and R¹⁰ are hydrogen, methyl or tert-butyl,
the radical R¹¹ is hydrogen, methyl or methoxy, and
the radicals R⁷, R⁸ and R⁹ are hydrogen or methyl.

3. The method according to claims 1 to 2, wherein the ruthenium complex (I) comprises ruthenium in the oxidation state +2 or +3 and has the general formula (IA)
[Ru(L)XₐY_{b}]ₚ Z_{(p·c)} (IA)
where
X is in each case independently a neutral monodentate ligand, where two ligands X may also be bonded to form a neutral bidentate ligand,
Y is in each case independently an anionic monodentate ligand having a charge of "-1",
where Y and X together may also be an anionic bidentate ligand having a charge of "-1",
Z is in each case independently a non-coordinating anion having a charge of "-1", where two ligands Z may also be bonded to form a non-coordinating anion having a charge of "-2",
a, b and c are each independently 0, 1, 2 or 3, and
p is 1 or 2,
with the provisos that
a + b + c equals 1, 2, 3, 4, 5 or 6, and
b and c are determined such that the ruthenium complex (IA) has a total charge of "0".

4. The method according to claims 1 to 3, wherein the ruthenium complex (I) is obtained by reacting ligand (II) with a Ru precursor complex (IV).

5. The method according to claims 1 to 4, wherein an ester of the general formula (III) is used as ester 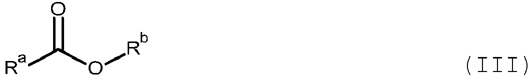 in which the radicals R^{a} and R^{b} are each independently a carbon-containing organic, linear or branched, noncyclic or cyclic, saturated or unsaturated, aliphatic, aromatic or araliphatic radical which is unsubstituted or interrupted or substituted by heteroatoms or functional groups and has a molar mass of 15 to 10 000 g/mol, where the two radicals R^{a} and R^{b} may also be bonded to each other.

6. The method according to claims 1 to 5, wherein the ruthenium complex (I) is formed in situ from a Ru precursor complex (IV) and ligand L (II).

7. The method according to claims 1 to 5, wherein the ruthenium complex (I) is formed in situ by reaction
(a) of an aldehyde or ketone of the general formula (Va) 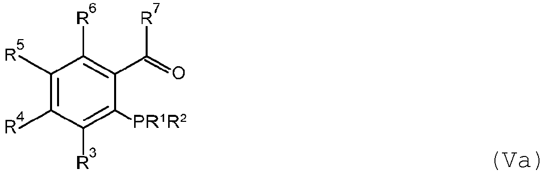 with an amine of the general formula (Vb) 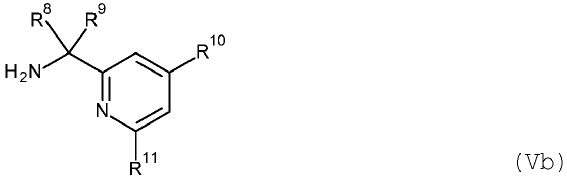 and/or
(b) of an amine of the general formula (VIa) 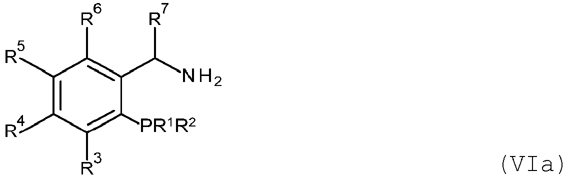 with an aldehyde or a ketone of the general formula (VIb) 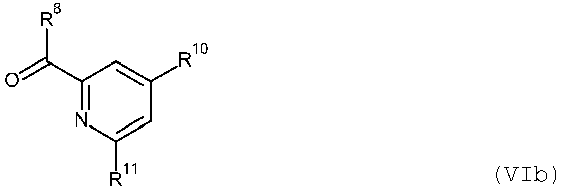 to give the ligand L (II) , where the radicals R¹ to R¹¹ each have the meaning defined above, and subsequent reaction of the ligand L (II) formed, without isolation or purification thereof, with a Ru precursor complex (IV) .

8. The method according to claims 1 to 7, wherein a molar ratio between the ester and the ruthenium complex (I) of 1 to 100 000 is used.

9. The method according to claims 1 to 8, wherein the hydrogenation is carried out in the presence of a base.

10. The method according to claim 9, wherein alkoxides or amides are used as base.

## Revendications

1. Procédé d'hydrogénation d'un ester avec de l'hydrogène moléculaire en alcools correspondants à une température de 50 à 200°C et à une pression de 0,1 à 20 MPa abs. en présence d'un complexe de ruthénium (I) pentacoordiné ou hexacoordiné, le complexe de ruthénium pouvant également être ponté en un dimère, **caractérisé en ce que** le complexe de ruthénium contient un ligand tridentate L présentant la formule générale (II) 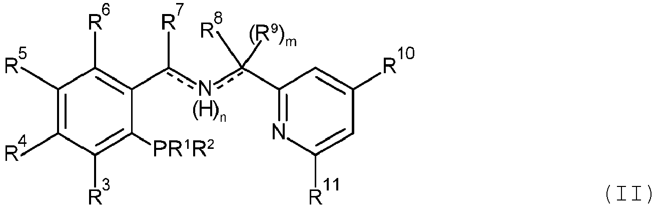 dans laquelle
R¹, R² représentent, indépendamment l'un de l'autre, un radical hydrocarboné aliphatique comprenant 1 à 8 atomes de carbone, un radical hydrocarboné aromatique comprenant 6 ou 10 atomes de carbone ou un radical hydrocarboné araliphatique comprenant 7 à 12 atomes de carbone, les radicaux hydrocarbonés mentionnés étant non substitués ou substitués par 1 à 3 groupes méthoxy, thiométhoxy ou diméthylamino et les deux radicaux R¹ et R² pouvant également être reliés l'un à l'autre avec formation d'un cycle de 5 à 10 chaînons, incluant l'atome de phosphore, R³, R⁴, R⁵, R⁶, R¹⁰, R¹¹ représentent, indépendamment les uns des autres, C₁-C₄-alkyle linéaire, C₃-C₄-alkyle ramifié, méthoxy, hydroxy, trifluorométhyle, nitrile ou dialkylamino comprenant, indépendamment les uns des autres, 1 à 4 atomes de carbone par groupe alkyle,
R⁷, R⁸, R⁹ représentent, indépendamment les uns des autres, hydrogène, C₁-C₄-alkyle linéaire ou C₃-C₄-alkyle ramifié,
n, m valent, indépendamment l'un de l'autre, 0 ou 1 et les doubles lignes continues-pointillées représentent une simple ou une double liaison, étant entendu que
| | |
|---|---|
| dans le cas de n = 1 | les deux doubles lignes continues-pointillées représentent une simple liaison et m vaut 1 et |
| dans le cas de n = 0 | une double ligne continue-pointillée représente une simple liaison et l'autre double ligne continue-pointillée représente une double liaison, où, dans le cas d'une double liaison sur le côté orienté vers le cycle phényle, m = 1, dans le cas d'une double liaison orientée vers le cycle pyridyle, m = 0, ou les deux doubles lignes continues-pointillées représentent une simple liaison et m vaut 1. |

2. Procédé selon la revendication 1, **caractérisé en ce qu'**on utilise un ligand L (II), dans lequel
(i) n et m valent à chaque fois 1 et les deux doubles lignes continues-pointillées représentent une simple liaison ou
(ii) n vaut 0 et m vaut 1 et la double ligne continue-pointillée orientée vers le cycle phényle représente une double liaison et la double ligne continue-pointillée orientée vers le cycle pyridyle représente une simple liaison
et
les deux radicaux R¹ et R² représentent phényle, p-toluyle, 3,5-diméthyl-4-méthoxyphényle, iso-butyle ou cyclohexyle,
les radicaux R³, R⁴ et R⁶ représentent hydrogène,
les radicaux R⁵ et R¹⁰ représentent hydrogène, méthyle ou tert-butyle,
le radical R¹¹ représente hydrogène, méthyle ou méthoxy et
les radicaux R⁷, R⁸ et R⁹ représentent hydrogène ou méthyle.

3. Procédé selon les revendications 1 à 2, **caractérisé en ce que** le complexe de ruthénium (I) contient le ruthénium dans l'étage d'oxydation +2 ou +3 et présente la formule générale (IA)
[Ru(L)XₐY_{b}]ₚ Z_{(p·c)} (Ia)
dans laquelle
X représente, à chaque fois indépendamment, un ligand monodentate neutre, deux ligands X pouvant également être reliés en un ligand bidentate neutre,
Y représente, à chaque fois indépendamment, un ligand monodentate anionique de charge "-1",
Y et X pouvant également représenter, conjointement, un ligand bidentate anionique de charge "-1",
Z représente, à chaque fois indépendamment, un anion non coordinant de charge "-1", deux ligands Z pouvant également être reliés en un anion non coordinant de charge "-2",
a, b et c valent, à chaque fois indépendamment les uns des autres, 0, 1, 2 ou 3 et
p vaut 1 ou 2,
étant entendu que
a+b+c vaut 1, 2, 3, 4, 5 ou 6 et
b et c sont déterminés de telle sorte que le complexe de ruthénium (IA) présente la charge totale "0".

4. Procédé selon les revendications 1 à 3, **caractérisé en ce qu'**on obtient le complexe de ruthénium (I) par transformation du ligand (II) avec un complexe précurseur de Ru (IV).

5. Procédé selon les revendications 1 à 4, **caractérisé en ce qu'**on utilise, comme ester, un ester de formule générale (III) 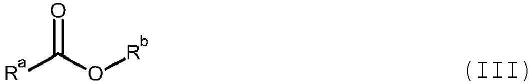 dans laquelle les radicaux R^{a} et R^{b} représentent, à chaque fois indépendamment l'un de l'autre, un radical organique contenant du carbone, linéaire ou ramifié, non cyclique ou cyclique, saturé ou insaturé, aliphatique, aromatique ou araliphatique, non substitué ou interrompu ou substitué par des hétéroatomes ou des groupes fonctionnels présentant une masse molaire de 15 à 10.000 g/mole, les deux radicaux R^{a} et R^{b} pouvant également être reliés l'un à l'autre.

6. Procédé selon les revendications 1 à 5, **caractérisé en ce qu'**on forme le complexe de ruthénium (I) in situ à partir d'un complexe précurseur de Ru (IV) et du ligand L (II).

7. Procédé selon les revendications 1 à 5, **caractérisé en ce qu'**on forme le complexe de ruthénium (I) in situ par transformation
(a) d'un aldéhyde ou d'une cétone de formule générale (Va) 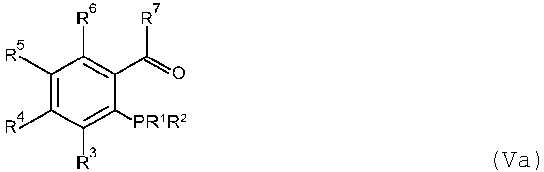 avec une amine de formule générale (Vb) 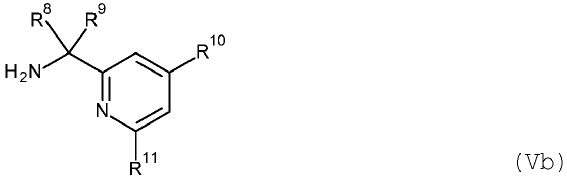 et/ou
(b) d'une amine de formule générale (VIa) 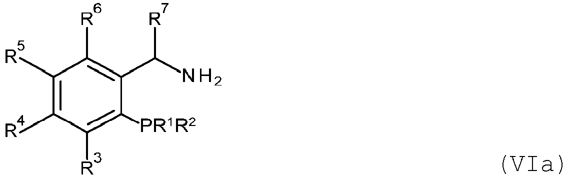 avec un aldéhyde ou une cétone de formule générale (VIb) 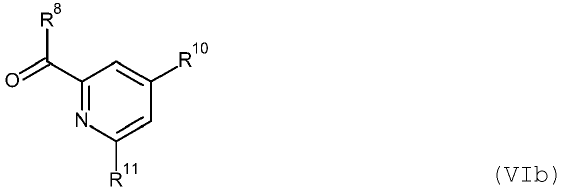 en ligand L (II), les radicaux R¹ bis R¹¹ présentant à chaque fois la signification définie ci-dessus, et par transformation consécutive du ligand L (II) formé sans son isolement ou sa purification avec un complexe précurseur de Ru (IV).

8. Procédé selon les revendications 1 à 7, **caractérisé en ce qu'**on utilise un rapport molaire entre l'ester et le complexe de ruthénium (I) de 1:100.000.

9. Procédé selon l'une des revendications 1 à 8, **caractérisé en ce qu'**on réalise l'hydrogénation en présence d'une base.

10. Procédé selon la revendication 9, **caractérisé en ce qu'**on utilise des alcoolates ou des amides comme base.
